# EUROPEAN PATENT APPLICATION

(11) **EP 4 382 111 A1**
(43) Date of publication of application: **12.06.2024**
(21) Application number: 22386089.1
(22) Date of filing: 09.12.2022
(51) Int. Cl.: A61K 35/545, A61P 9/00, A61K 35/12, A61K 35/44, A61L 27/54, A61P 9/10, A61P 9/12, A61P 11/00, A61P 17/02, B01D 15/34

(54) **EXTRACELLULAR VESICLES THAT PROMOTE ANGIOGENESIS OR NEOVASCULARISATION**

(71) Applicant: The University Court Of The University of Edinburgh, Edinburgh EH8 9YL (GB)
(72) Inventor: Kesidou, Despoina, Edinburgh, EH16 4SB (GB); Beqqali, Abdelaziz, Edinburgh, EH16 4TJ (GB); Baker, Andrew, Edinburgh, EH16 4TJ (GB); Brittan, Mairi, Edinburgh, EH16 4TJ (GB); Mountford, Joanne, Edinburgh, EH14 4BE (GB)
(74) Representative: Marks & Clerk LLP

(57) **Abstract**

The present disclosure relates to extracellular vesicles (EVs) (and their contents) from a pluripotent stem-cell derived endothelial cell product (ECP); as well as their isolation; uses and methods employing the EVs in therapy, such as in promoting angiogenesis and/or neovascularisation.

## Description

### Field

The present disclosure relates to extracellular vesicles (EVs) (and their contents) from a pluripotent stem-cell derived endothelial cell product (ECP); as well as their isolation; uses and methods employing the EVs in therapy, such as in promoting angiogenesis and/or neovascularisation.

### Background

Cardiovascular disease (CVD) remains the most common cause of mortality worldwide with the World Health Organisation (WHO) reporting that in 2017, 17.9 million people died from CVDs. Of those deaths, an estimated 7.4 million were attributed to coronary heart disease (CHD) *alone*(*WHO-Cardiovascular Diseases (CVDs),* 2017). CHD is characterised by narrowing of the coronary arteries due to the gradual formation and the subsequent rupture of plaque within the vessel walls. Blockage of these arteries results in oxygen and nutrient deprivation of the downstream tissue. Consequently, ischaemic damage and cardiomyocyte death occur in the affected region of the heart, a phenomenon known as myocardial infarction (MI)(Thygesen et al., 2019). Therapeutic neovascularisation has been proposed as a possible strategy to create new myocardial blood vessel networks and reduce the extent of cardiomyocyte damage. Paracrine cell communication plays a critical role in the control of this process (Gnecchi et al., 2008). Cell communication in a paracrine manner is regulated by several mechanisms, including extracellular vesicles (EVs). EVs carry and transfer various bioactive molecules, such as small non-coding RNAs, proteins, and lipids, that regulate signalling pathways in the recipient cells (Théry et al., 2018).

Despite some controversy, preclinical studies have demonstrated that EVs hold promise in the regulation of complex processes, such as post-ischaemic neovascularisation (Kesidou et al., 2020). However, the optimal source of such EVs, their respective cargo and function and their translation to clinical opportunities remains in its relative infancy.

Endothelial cell (EC) injury and activation are important factors in the cellular release of EVs. In general, EC-EVs are present at lower concentrations under physiological conditions and, upon activation, are released at higher levels from ECs (Koga et al., 2005). Circulating EVs released from ECs have been shown to play a role in activating vascular ECs (Ridger et al., 2017). Emerging evidence, however, suggests that EVs of endothelial origin may play a versatile role in neovascularisation since their effect depends not only on the EV-donor cells but also on the dose or number of EVs that the recipient cell is exposed to (Lacroix et al., 2007). Nonetheless, a potential role of the endothelial secretome in EC activation and neovascularisation has been proposed by EC-transplantation studies that show improvements in capillary density and blood flow post EC-transplantation, despite limited retention to the ischaemic tissues (Chekanov et al., 2003). We have previously shown that transplantation of human embryonic stem cell derived EC products (hESC-to-ECPs) following left femoral artery occlusion resulted in increased capillary density in mice at 21 days post-ischemia. Further, we have shown improved cardiac function after cell injection into the heart after myocardial ischemia (Ana-Mishel Spiroski et al., 2022), demonstrating the broad relevance of these cells as a therapy. Despite their ability to increase capillary density, these cells showed poor retention (MacAskill et al., 2018), suggesting that acute paracrine mechanisms of action may be involved. These findings were in line with previous cell transplantation studies, which have revealed that only very few of the transplanted cells can engraft to the site of injury (Tompkins et al., 2018).

### Summary

Since the increased neovascularisation rates post-hESC-ECP transplantation implies a paracrine mechanism of action, here, we sought to investigate the effect of hESC-ECP-derived EVs (hESC-eEVs) in angiogenesis. As described herein, we show that hESC-eEVs at very low concentrations promote EC tube formation and wound healing and are enriched in known angiogenic and other miRNAs with non-reported roles in angiogenesis. Moreover, we demonstrated that hESC-eEVs induce angiogenesis at low doses and that may be due to selected miRNA molecules which are present in the hESC-eEVs, as well as other components that are present in the EVs.

In a first aspect there is provided an isolated population of pro-angiogenic and/or pro-neovasculogenic extracellular vesicles (EVs) and/or the contents thereof, obtainable from pluripotent stem-cell derived endothelial cell products (ECPs).

There is also provided an isolated population of pro-angiogenic and/or pro-neovasculogenic EVs and/or their contents thereof, obtainable from pluripotent stem-cell derived ECPs for use in therapy, such as for use in promoting angiogenesis and/or neovascularisation, in a subject in need thereof. In one teaching the isolated population of pro-angiogenic and/or pro-neovasculogenic EVs and/or their contents thereof is obtained from pluripotent stem-cell derived ECPs.

There is also provided a method of treatment, such as by promoting angiogenesis and/or neovascularisation, comprising administering to a subject in need thereof an isolated population of pro-angiogenic and/or pro-neovasculogenic EVs and/or their contents thereof, obtained from pluripotent stem-cell derived ECPs.

In one embodiment, the present disclosure is directed to the use of intact EVs as described herein, Angiogenesis primarily refers to the formation of new blood vessels from pre-existing blood vessels, while neovascularisation is the process of *de novo* formation of blood vessels or formation of new blood vessels from pre-existing blood vessels. The present disclosure may relate to both angiogenesis and/or neovascularisation. That is, the present disclosure may relate to promoting angiogenesis alone, promoting neovascularisation alone, or promoting both angiogenesis and neovascularisation. The remaining description may refer to either term alone, but unless the context dictates otherwise, reference to angiogenesis is to be understood to extend to neovascularisation and vice versa.

The terms "therapy" or "treatment", as used herein, may be understood to relate to prevention and/or treatment of a condition or a disease.

As used herein the phrase "pluripotent stem cells" refers to cells , which are capable of differentiating into cells of all three embryonic germ layers (i.e., endoderm, ectoderm and mesoderm). The phrase "pluripotent stem cells" includes embryonic stem cells (ESCs) and/or induced pluripotent stem cells (iPS cells).

The phrase "embryonic stem cells" as used herein refers to cells which are obtained from the embryonic tissue formed after gestation (e.g., blastocyst) before implantation (i.e., a preimplantation blastocyst); extended blastocyst cells (EBCs) which are obtained from a post-implantation/pre-gastrulation stage blastocyst (see WO2006/040763]; and/or embryonic germ (EG) cells which are obtained from the genital tissue of a fetus any time during gestation, preferably before 10 weeks of gestation.

According to some embodiments, the pluripotent stem cells are embryonic stem cells, such as from a human or primate (e.g., monkey) origin. Embryonic stem cells can be obtained using well-known cell-culture methods. For example, human embryonic stem cells can be isolated from human blastocysts. Human blastocysts are typically obtained from human *in-vivo* preimplantation embryos or from *in-vitro* fertilized (IVF) embryos. Alternatively, a single cell human embryo can be expanded to the blastocyst stage. For the isolation of human ES cells the zona pellucida is removed from the blastocyst and the inner cell mass (ICM) is isolated by immunosurgery, in which the trophectoderm cells are lysed and removed from the intact ICM by gentle pipetting. The ICM is then plated in a tissue culture flask containing the appropriate medium which enables its outgrowth. Following 9 to 15 days, the ICM derived outgrowth is dissociated into clumps either by a mechanical dissociation or by an enzymatic degradation and the cells are then re-plated on a fresh tissue culture medium. Colonies demonstrating undifferentiated morphology are individually selected by micropipette, mechanically dissociated into clumps, and re-plated. Resulting ES cells are then routinely split every 4-7 days. For further details on methods of preparation human ES cells see Thomson et al., [U.S. Pat. No. 5,843,780; Science 282: 1145, 1998; Curr. Top. Dev. Biol. 38: 133, 1998; Proc. Natl. Acad. Sci. USA 92: 7844, 1995]; Bongso et al., [Hum Reprod 4: 706, 1989]; and Gardner et al., [Fertil. Steril. 69: 84, 1998].

It will also be appreciated that commercially available stem cells can also be employed. Human ES cells can be purchased from commercial sources including the NIH human embryonic stem cells registry (www://escr(dot)nih(dot)gov). Non-limiting examples of commercially available embryonic stem cell lines are BG01, BG02, BG03, BG04, CY12, CY30, CY92, CY10, TE03, TE04 and TE06.

The phrase "induced pluripotent stem (iPS) cell" (or embryonic-like stem cell) as used herein refers to a proliferative and pluripotent stem cell which is obtained by de-differentiation of a somatic cell (e.g., an adult somatic cell). According to some embodiments, an iPS cell is characterized by a proliferative capacity which is similar to that of ESCs and thus can be maintained and expanded in culture for an almost unlimited time.

iPS cells can be endowed with pluripotency by genetic manipulation which re-program the cell to acquire embryonic stem cells characteristics. For example, the iPS cells of the invention can be generated from somatic cells by induction of expression of Oct-4, Sox2, Kfl4 and c-Myc in a somatic cell essentially as described in Takahashi and Yamanaka, 2006, Takahashi et al, 2007, Meissner et al, 2007, and Okita K., et al, 2007, Nature 448: 313-318). Additionally or alternatively, the iPS cells of the invention can be generated from somatic cells by induction of expression of Oct4, Sox2, Nanog and Lin28 essentially as described in Yu et al, 2007, and Nakagawa et al, 2008. It should be noted that the genetic manipulation (re-programming) of the somatic cells can be performed using any known method such as using plasmids or viral vectors, or by derivation without any integration to the genome [Yu J, et al., Science. 2009, 324: 797-801].

The iPS cells can be obtained by inducing de-differentiation of embryonic fibroblasts [Takahashi and Yamanaka, 2006; Meissner et al, 2007], fibroblasts formed from hESCs [Park et al, 2008], Fetal fibroblasts [Yu et al, 2007; Park et al, 2008], foreskin fibroblast [Yu et al, 2007; Park et al, 2008], adult dermal and skin tissues [Hanna et al, 2007; Lowry et al, 2008], b-lymphocytes [Hanna et al 2007] and adult liver and stomach cells [Aoi et al, 2008].

iPS cell lines are also available via cell banks such as the WiCell bank. Non-limiting examples of commercially available iPS cell lines include the iPS foreskin clone 1 [WiCell Catalogue No. iPS(foreskin)-1-DL-1], the iPSIMR90 clone 1 [WiCell Catalogue No. iPS(IMR90)-1-DL-1], and the iPSIMR90 clone 4 [WiCell Catalogue No. iPS(IMR90)-4-DL-1].

According to some embodiments, iPS cells are human induced pluripotent stem cells.

The term "pluripotent stem-cell derived ECPs" as used herein is understood to refer to cell products derived from differentiating or partially differentiating pluripotent stem cells and which comprises at least 40%, 50%, 60% or 70% endothelial cells. The hESC-derived ECP may be a mixed population comprised of endothelial cells, and other differentiated cell types including those typically expressing markers indicative of mesenchymal or pericyte lineages (see for example MacAskill et al., 2018).

As used herein, the term "population of extracellular vesicles" refers to a population of extracellular vesicles having a pro-angiogenic and/or pro-neovasculogenic characteristic(s) as a result of being produced from pluripotent stem-cell derived ECPs. The terms "population of extracellular vesicles" and "extracellular vesicles" can be used interchangeably to refer to a population of extracellular vesicles having a pro-angiogenic and/or pro-neovasculogenic characteristic(s).

As used herein, the term "extracellular vesicles", abbreviated as EVs, encompass exosomes. The terms "extracellular vesicles" and "EVs," as used herein, may in some embodiments refer to a membranous particle having a diameter (or largest dimension where the particles is not spheroid) of between about 10 nm to about 5000 nm, more typically between 30 nm and 1000 nm, and most typically between about 50 nm and 750 nm. Most commonly, EVs will have a size (average diameter) that is up to 5% of the size of the donor cell. Therefore, especially contemplated EVs include those that are shed from a cell.

As used herein, an isolated EV (or population of EVs) is one which is physically separated from its natural environment. An isolated EV may be physically separated, in whole or in part, from the pluripotent stem-cell derived ECPs secreting the EVs. In some embodiments of the disclosure, a composition of isolated extracellular vesicles may be free of the ECPs from which they have been produced. In some embodiments, the EVs may be present in, or may be free or substantially free from media used to culture the ECPs. In some embodiments, the isolated EVs may be provided at a higher concentration than EVs present in the media from which they have been derived.

The extracellular vesicles (EVs) of the disclosure can be obtained from ECPs, which have been derived from pluripotent stem cells. Thus, the pluripotent stem cells have been directed to differentiate into endothelial cell products using appropriate techniques known in the art [see for example (Ana-Mishel Spiroski et al., 2022; MacAskill et al., 2018; McCracken et al., 2019; Zhang et al., 2017)].

Typically, suitable endothelial cells may be characterised as expressing one or more (ideally both) of cell surface markers CD31 and CD144. Conveniently, at least 40%, 50%, or 60% of cells derived from pluripotent stem cells, co-express CD31 and CD144. Additionally, typically less than 10%, 5%, 2.5%,1% of ECPs express the pluripotency markers SSEA-3/TRA-1-60.

Generally, any suitable method for isolating, purifying and/or enriching EVs can be used, such as methods comprising magnetic particles, filtration, dialysis, ultracentrifugation, ExoQuick^{™} (Systems Biosciences, CA, USA), and/or chromatography. In some embodiments, extracellular vesicles are isolated by centrifugation and/or ultracentrifugation. EVs can also be purified by ultracentrifugation of clarified conditioned media. They can also be purified by ultracentrifugation into a sucrose cushion. The protocol is described in, for example, Thery et al. Current Protocols in Cell Biol. (2006) 3.22, which is incorporated herein by reference (Théry et al., 2006). In some embodiments, extracellular vesicles are isolated by step size exclusion chromatography. The protocol is described in, for example, Being et al. Journal of Extracellular Vesicles (2014) 3:23430, which is incorporated herein by reference. See also Paganini, C., Capasso Palmiero, U., Pocsfalvi, G., Touzet, N., Bongiovanni, A. and Arosio, P. (2019), Scalable Production and Isolation of Extracellular Vesicles: Available Sources and Lessons from Current Industrial Bioprocesses. Biotechnol. J., 14: 1800528 and Thanaporn Liangsupree, Evgen Multia, Marja-Liisa Riekkola, Modern isolation and separation techniques for extracellular vesicles, Journal of Chromatography A, Volume 1636, 2021, for examples of other protocols, which may be followed

A detailed method for harvest of EVs from ECPs derived from pluripotent stem cells comprises, consists essentially of, or consists of, a combination of centrifugation, ultrafiltration and size exclusion chromatography (SEC). A detailed protocol is provided in the Examples.

Thus, in a further aspect, there is provided a method of isolating EVs from ECPs derived from pluripotent stem cells, the method comprising:
a) subjecting a fluid comprising EVs produced by endothelial cell products derived from pluripotent stem cells to centrifugation and/or ultrafiltration, in order to separate EVs from larger molecular weight material and obtain an EVs enriched fluid; and
b) subjecting the EVs enriched fluid to size-exclusion chromatography in order to obtain fluid fractions comprising isolated EVs.

The fluid comprising EVs produced by ECPs derived from pluripotent stem cells is conditioned media ( conditioned media, is media which has been exposed to cells for a certain amount of time, for example 12 - 36 hours, e.g. 24hours. This media contains cell-secreted factors, including extracellular vesicles) from which the ECPs have been removed by centrifugation. Centrifugation may be conducted so as to remove cells and cell debris from the media but retain EVs in the media.

Ultrafiltration may comprise centrifugation through a filter of a suitable molecular weight, such as 100KDa, in order to separate lower molecular weight material from the EVs.

Size exclusion chromatography (SEC) separates molecules based on their size by filtration through a resin. The resin consists of spherical beads containing pores of a specific size. Separation occurs when molecules of different sizes are included or excluded from the pores within the matrix. Small molecules diffuse into the pores and their flow through the column is retarded according to their size, while large molecules do not enter the pores and are eluted in the column's void volume. Consequently, molecules separate based on their size as they pass through the column and are eluted in order of decreasing molecular weight (MW). Operating conditions and gel selection depend on the application and the desired resolution. Exemplary SEC media include sephacryl, sephadex, superose, superdex and sepharose.

There is further provided an isolated population of pro-angiogenic and/or pro-neovasculogenic extracellular vesicles (EVs) and/or their contents thereof prepared according to a method as described herein, for use in promoting angiogenesis and/or neovascularisation, as described herein.

A suitable isolated population of pro-angiogenic EVs may be determined as being positive for the markers CD63 and CD81 and negative for cellular contamination marker calnexin. Moreover, the isolated population of pro-angiogenic EVs is desirably free of non-EV protein contaminants.

The angiogenic activity of EVs may be determined using known techniques (Irvin et al., 2014). For example, EVs may be applied to a tube formation and/or wound healing assay, as described herein. Other suitable assays also include *in-vitro* proliferation assays but also ex-*vivo* and *in-vivo* assays such as aortic ring assay, chorioallantoic membrane assay (CAM), Matrigel plug assay, fluorescent zebrafish assay, dorsal air sac model and chamber assay.

A disadvantage of pro-angiogenic EVs previously known in the art is that in order to provide their angiogenic effect, the pro-angiogenic EVs need to be employed at high dose. Advantageously, the pro-angiogenic and/or pro-neovasculogenic EVs of the present disclosure have been observed to exert their pro-angiogenic and/or pro-neovasculogenic effect at much lower doses (typically less than 50, 25, 10, 5, such as low as 1 EV/cell). Moreover, increasing the EV-dose does not increase the angiogenic effect. This demonstrates that the pro-angiogenic and/or pro-neovasculogenic EVs of the present disclosure are clearly different to the pro-angiogenic and/or pro-neovasculogenic EVs previously known in the art.

EVs can be used immediately or stored, whether short term or long term, such as in a cryopreserved state, prior to use. Proteinase inhibitors are typically included in freezing media as they provide extracellular vesicle integrity during long-term storage. Freezing at -20°C is not preferable since it is associated with increased loss of extracellular vesicle activity. Quick freezing at -80°C is more preferred as it preserves activity. See for example Kidney International (2006) 69, 1471-1476, which is incorporated herein by reference (Zhou et al., 2006). Additives to the freezing media may be used in order to enhance preservation of extracellular vesicle biological activity. Such additives will be similar to the ones used for cryopreservation of intact cells and may include, but are not limited to DMSO, glycerol and polyethylene glycol.

The present disclosure further relates to a pharmaceutical composition comprising an isolated population of pro-angiogenic and/or pro-neovasculogenic EVs and/or their contents thereof, obtainable, or obtained from pluripotent stem-cell derived endothelial cell products. Preferably, the pharmaceutical composition comprises an isolated population of pro-angiogenic and/or pro-neovasculogenic EVs and/or their contents thereof, obtainable, or obtained from pluripotent stem-cell derived ECPs together with a pharmaceutically acceptable carrier or excipients. The composition may comprise carriers and excipients, as would be well known to someone of skill in the art. Such compositions may find use in the promotion of angiogenesis and/or neovascularisation.

The isolated population of pro-angiogenic and/or pro-neovasculogenic EVs and/or their contents thereof as described herein, as well as compositions may find use in the treatment of ischemic tissue disorders, cardiovascular diseases such as pulmonary hypertension and/or wound healing for example. As used herein, the term ischemic tissue disorders includes disorders and complications which include cardiovascular ischemic disorders including post-myocardial infarction cardiac injury and progression to heart failure and lower limb ischemia, mesenteric ischemic disorders, and/or renovascular ischemic disorders.

The isolated population of pro-angiogenic and/or pro-neovasculogenic EVs and/or their contents thereof as described herein, as well as compositions, may find use in treatments where new/improved vascularisation would be beneficial in tissue transplant therapy and/or graft engraftment, for example.

In a further embodiment, the disclosure extends to a medical device coated with an isolated population of pro-angiogenic and/or pro-neovasculogenic EVs and/or their contents thereof as described herein, or a composition as described herein. The medical device may be a stent, a suture, a bandage, a dressing, a prosthesis, biomaterials engineered for wound healing and the like.

The term "ischemic" and cardiovascular" disorders and diseases, as used herein may include an interruption of the blood supply to an organ or tissue. An ischemic event may often be the result of a blood clot and in patients with atherosclerotic stenosis is most often caused when emboli dislodge from the atherosclerotic lesion. The resulting stenosis, or narrowing or blockage of an artery or other vessel due to this obstruction may result in a large number of adverse conditions, many of which have severe consequences for the subject. Ischemic and cardiovascular disorders/diseases as referred to herein include, but are not limited to stroke/transient ischemic attack or cerebrovascular attack, myocardial infarction, myocardial ischemia (angina pectoris), any cardiomyopathy complicated by myocardial ischemia (for instance symptomatic aortic stenosis, HOCM), cerebral bleeding, peripheral (unstable) angina pectoris, peripheral atherosclerotic artery disease and other major abnormalities occurring in the blood vessels. The term "abnormalities occurring in the blood vessels" includes reference to coronary and cerebrovascular events as well as to peripheral vascular disease. The term "ischemic cardiovascular or cerebrovascular event" is often the acute stage of a medical condition that is broadly encompassed by the term "cardiovascular, cerebrovascular and peripheral artery disease" (here collectively termed "cardiovascular disease"). Such diseases include cerebrovascular and also peripheral artery diseases.

The term "ischemia", as used herein, refers to an absolute or relative shortage of the blood supply or an inadequate flow of blood to an organ, body part or tissue. Relative shortage refers to the discrepancy between blood supply (oxygen delivery) and blood request (oxygen consumption by tissue). The restriction in blood supply, generally due to factors in the blood vessels, is most often, but not exclusively, caused by constriction or blockage of the blood vessels by thromboembolism (blood clots) or atherosclerosis (lipid-laden plaques obstructing the lumen of arteries). Ischemia result in damage or dysfunction of tissue. Ischemia of the heart muscle results in angina pectoris, and is herein referred to as ischemic heart disease.

The term "cardiovascular disease" (CVD) generally refers to a number of diseases that affect the heart and circulatory system, including aneurysms; angina; arrhythmia; atherosclerosis; cardiomyopathies; cerebrovascular accident (stroke); cerebrovascular disease; congenital heart disease; congestive heart failure; coronary heart disease (CHD), also referred to as coronary artery disease (CAD), ischemic heart disease or atherosclerotic heart disease; dilated cardiomyopathy; diastolic dysfunction; endocarditis; heart failure; hypertension (high blood pressure); hypertrophic cardiomyopathy; myocardial infarction (heart attack); myocarditis; peripheral vascular disease; small vessel disease; and venous thromboembolism. As used herein, the term "cardiovascular disease" also encompasses reference to ischemia; arterial damage (damage to the endothelial lineage) due to physical damage (endartiectomie, balloon angioplasty) or as a result of chronic damage (including atherosclerosis); myocardial damage (myocardial necrosis); and myonecrosis. In general, any physiological or pathophysiological condition that elicits an angiogenic or neovascularisation response is encompassed by the term "cardiovascular disease" as used herein.

Unless otherwise specified, "a" or "an" means "one or more."

Unless specifically defined otherwise, all technical and scientific terms used herein shall be taken to have the same meaning as commonly understood by one of ordinary skill in the art.

As used herein, the term "subject" (also referred to herein as a "patient") includes warm-blooded animals, preferably mammals, including humans. In a preferred embodiment, the subject is a primate. In an even more preferred embodiment, the subject is a human.

As used herein the terms "treating", "treat," or "treatment" include reducing, mitigating, or eliminating at least one symptom of a disease or condition.

As used herein the terms "preventing", "prevent" or "prevention" include stopping or hindering the appearance or existence of at least one symptom of a disease or condition. Alternatively, the terms "preventing", "prevent" or "prevention" may include stopping or hindering the appearance or existence of at least one symptom of a disease or condition.

### Detailed description

The present disclosure will now be further described by way of examples and with reference to the figures, which show:

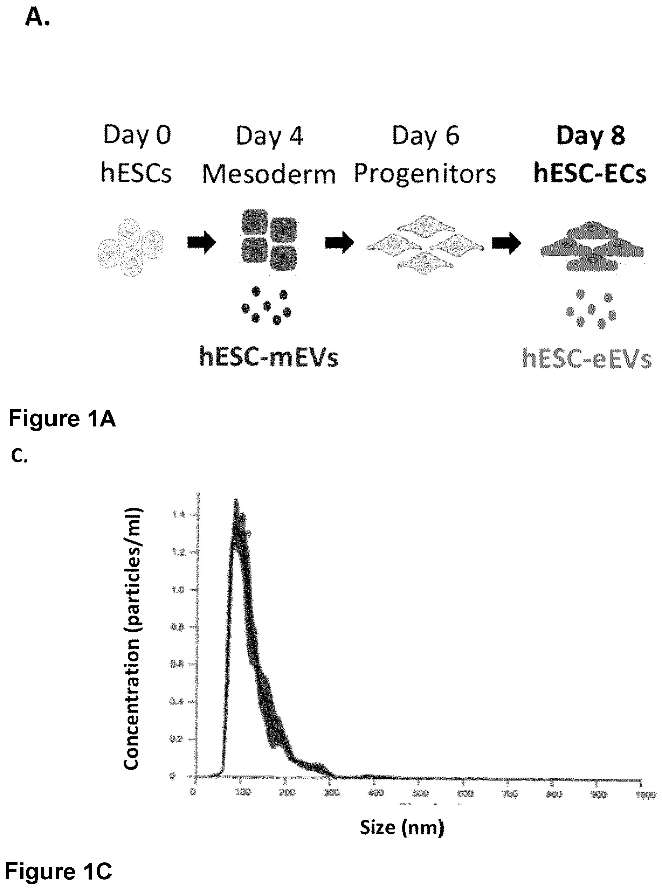

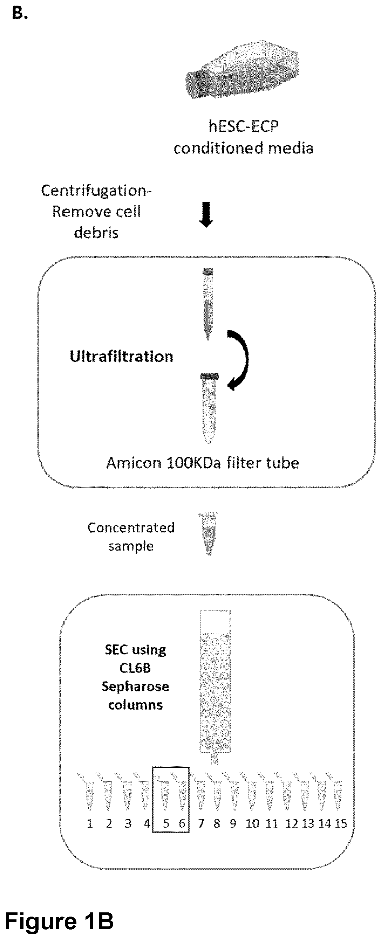

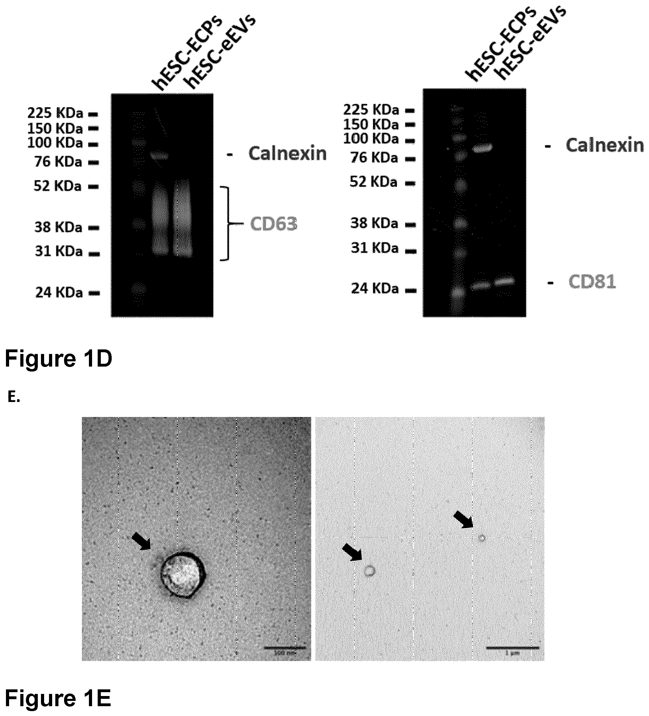

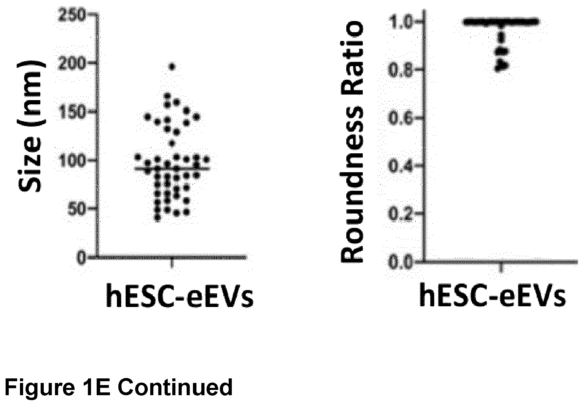

**Figure 1****. Isolation and characterization of EVs from hESC-ECP conditioned media. A. Schematic representation of hESC-ECP differentiation and EV secretion from cells at the mesodermal (day 4) and endothelial (day 8) stage.** B. Workflow of particle isolation from hESC-ECP conditioned media by a combination of ultrafiltration with SEC. C. Representative graph from NTA showing the size distribution of EVs in the pooled fractions 5 and 6. D. EV surface marker characterisation of the pooled fractions 5 and 6 by Western blot. Cell lysates were used as a positive control. Detection of calnexin emerges as a band at 90KDa, detection of CD63 emerges at 30-65KDa and detection of CD81 emerges at 22-26KDa. E. TEM imaging of particles in the pooled fractions 5 and 6. The arrows indicate EVs. Scale bar=100nm (left picture) and scale bar=1µm (right picture). Scatter plots represent the EV size distribution and roundness ratio (scale: 0-1) as determined by a total of 40 TEM images using the "TEM Exosome Analyzer" tool.

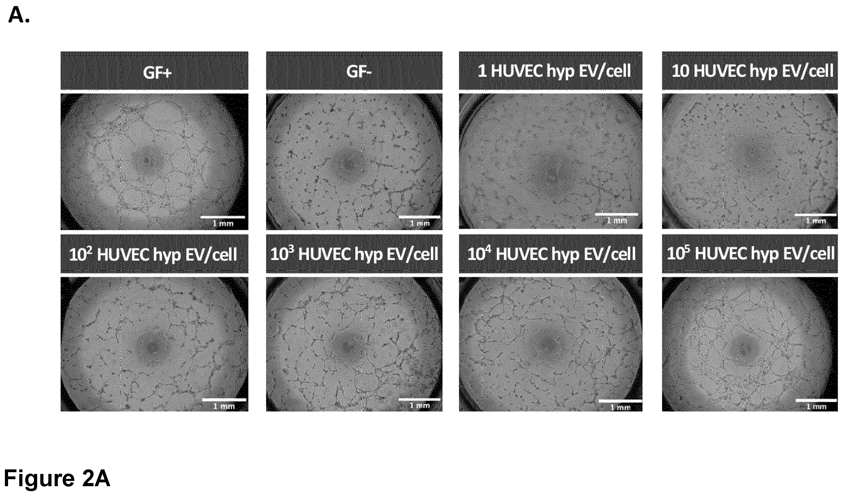

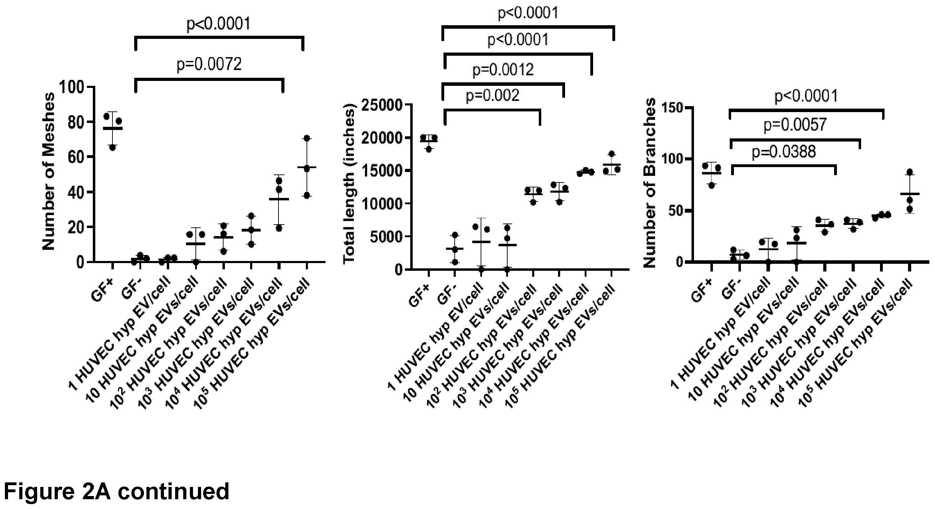

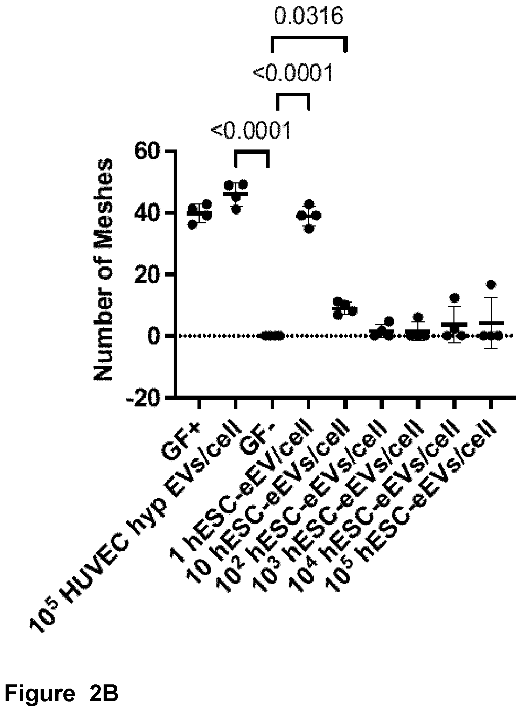

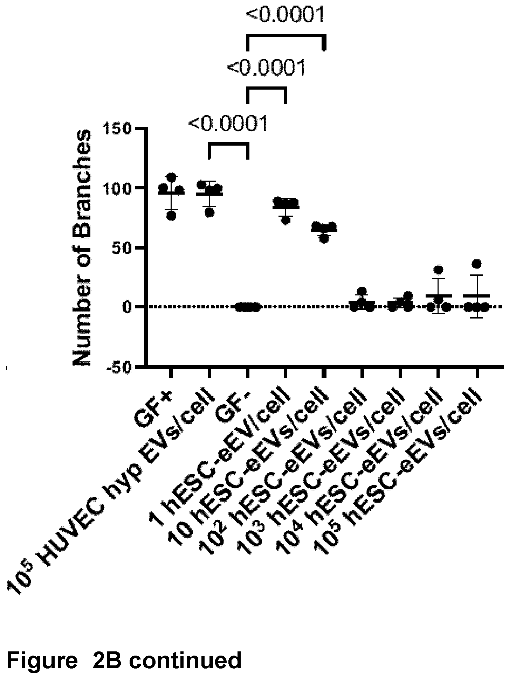

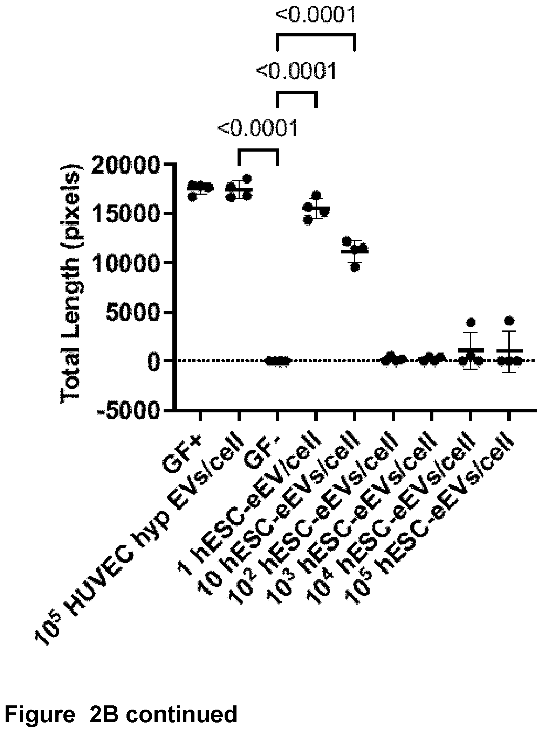

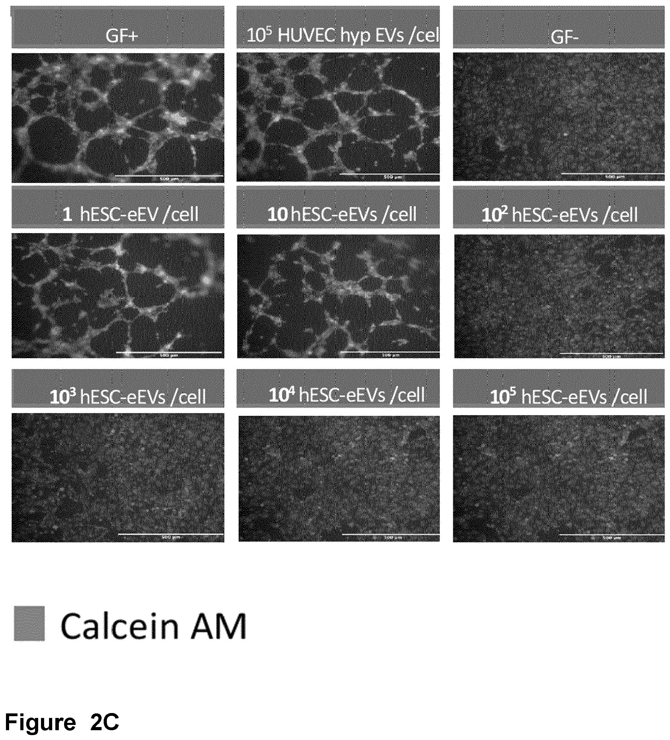

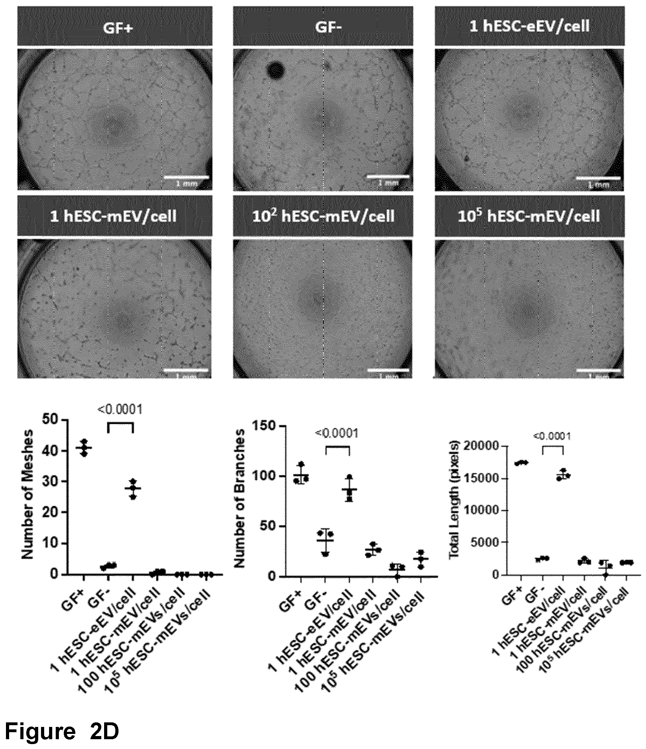

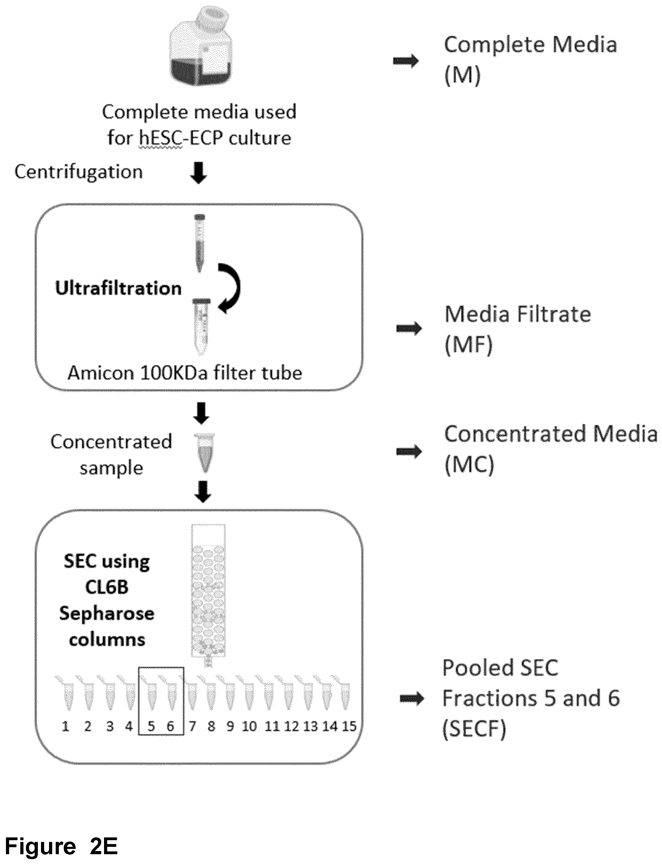

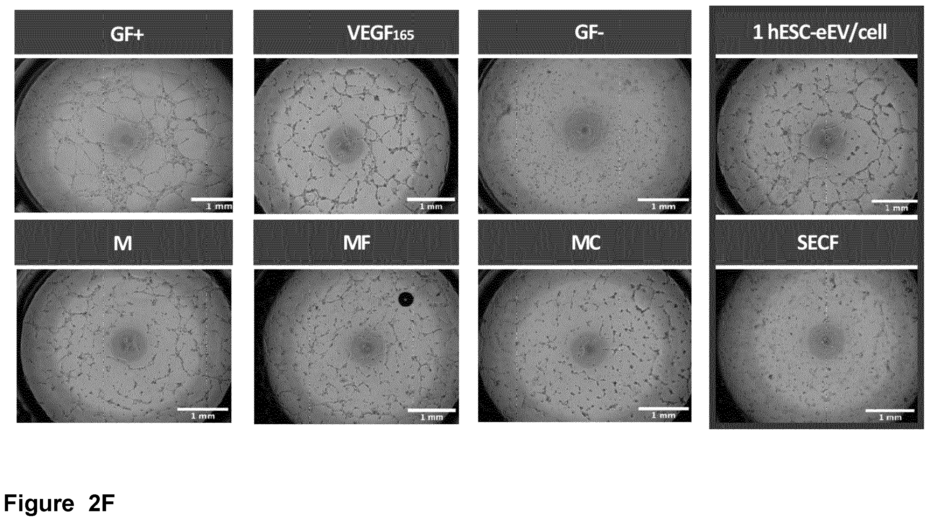

**Figure 2****. hESC-eEVs induce EC tube formation at low concentrations (<100 EVs/cell) A.** Tube Formation Assay on HCMECs using increasing concentrations of HUVEC hypoxic EVs (dose range: 1 - 10⁵ EVs/cell, n=3). Cells in fully supplemented media served as positive control (GF+). Cells cultured in basal media and treated with sterile filtered DPBS+P/S (vehicle control) served as negative control (GF-). Graphs (right) represent the number of meshes, branches and the total length of the tubes formed at 4h. Images were analysed using the "Angiogenesis Analyzer" tool on ImageJ. Black circles correspond to the control samples (GF+ and GF-) and purple triangles correspond to HUVEC hypoxic EV treated samples (HUVEC hyp EVs). Statistical significance (indicated with p-values) was determined by one-way ANOVA with Dunnett's multiple comparisons test. Error bars represent the SD. Scale bar=1mm. **B.** Quantification of Tube Formation Assay on HCMECs using increasing concentrations of hESC-eEVs (dose range: 1 - 10⁵ EVs/cell) (n=4). Cells in fully supplemented media (GF+) or treated with 10⁵ HUVEC hypoxic EVs/cell served as positive control. Cells cultured in basal media and treated with sterile 0.1µm filtered DPBS+P/S (vehicle control) served as negative control (GF-). Graphs represent the number of meshes, branches and the total length of the tubes formed at 4h. Data were analysed using the "Angiogenesis Analyzer" tool. Black circles correspond to the control samples (GF+ and GF-), purple triangles correspond to HUVEC hypoxic EV treated samples (HUVEC hyp EVs) and green circles correspond to hESC-eEV-treated samples. Statistical significance (indicated with p-values) was determined by one-way ANOVA with Dunnett's multiple comparisons test. Error bars represent the SD. **C.** Calcein AM staining of Tube Formation Assay on HCMECs using increasing concentrations of hESC-eEVs (dose range: 1 - 10⁵ EVs/cell, n=4). Cells in fully supplemented media (GF+) or treated with 10⁵ HUVEC hypoxic EVs/cell served as positive control. Cells cultured in basal media and treated with sterile 0.1µm filtered DPBS+P/S (vehicle control) served as negative control (GF-). Scale bar=500µm. **D.** Tube Formation Assay on HCMECs treated with hESC-mEVs (dose range: 1 - 10⁵ EVs/cell, n=3). Cells in fully supplemented media or treated with 1 hESC-eEV/cell served as positive control (GF+). Cells cultured in basal media and treated with sterile filtered DPBS+P/S (vehicle control) served as negative control (GF-). Images were analysed using the "Angiogenesis Analyzer" tool on ImageJ. Black circles correspond to the control samples (GF+ and GF-), green circles correspond to hESC-eEV-treated samples and red circles correspond to hESC-mEV-treated samples. Statistical significance (indicated with p-values) was determined by one-way ANOVA with Dunnett's multiple comparisons test. Graphs (right) represent the number of meshes, branches and the total length of tubes formed at 4h. Error bars represent the SD. Scale bar=1 mm. **E.** Schematic representation of the samples collected at different stages of the particle isolation from hESC-ECP cell culture media. **F.** Tube formation assay using samples collected at different stages of the particle isolation from hESC-ECP cell culture media. Cells in fully supplemented media (GF+) or treated with human recombinant VEGFA₁₆₅, or cultured with basal media and treated with one hESC-eEV/cell served as positive controls. Cells cultured in basal media and treated with DPBS+P/S (vehicle control) served as negative control (GF-).

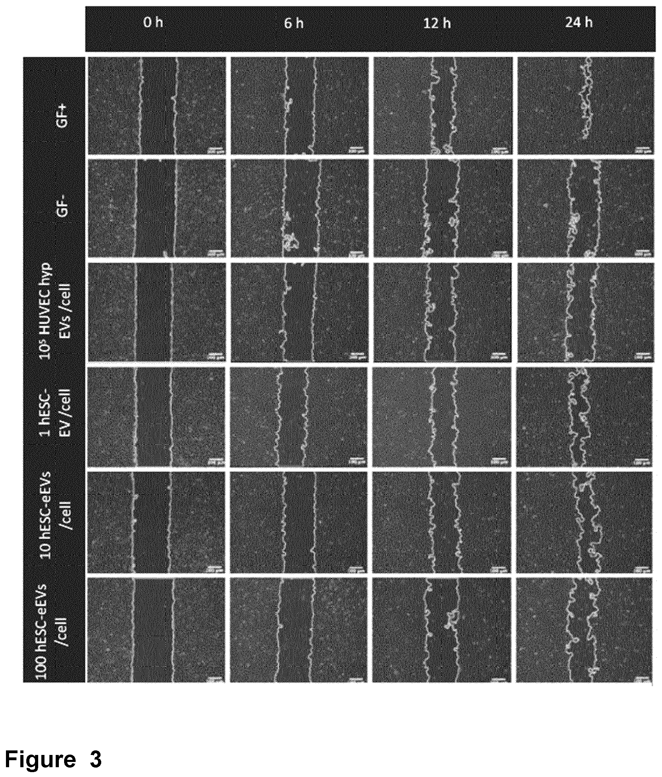

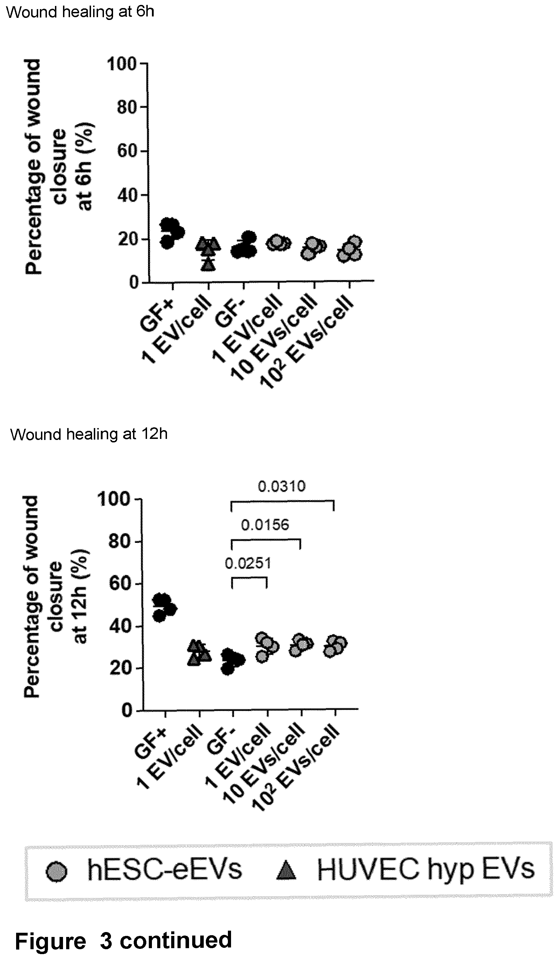

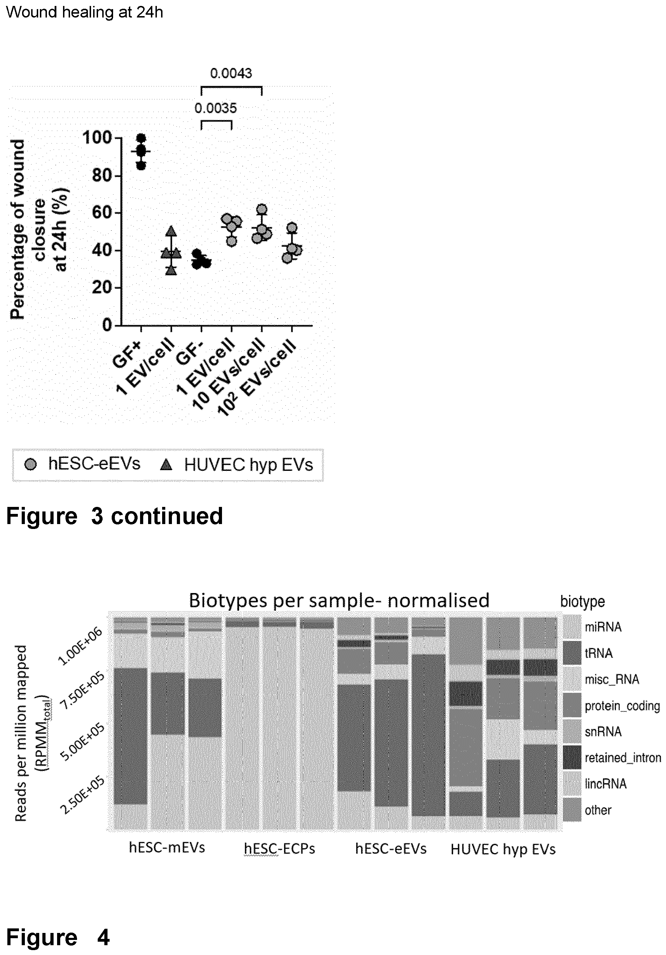

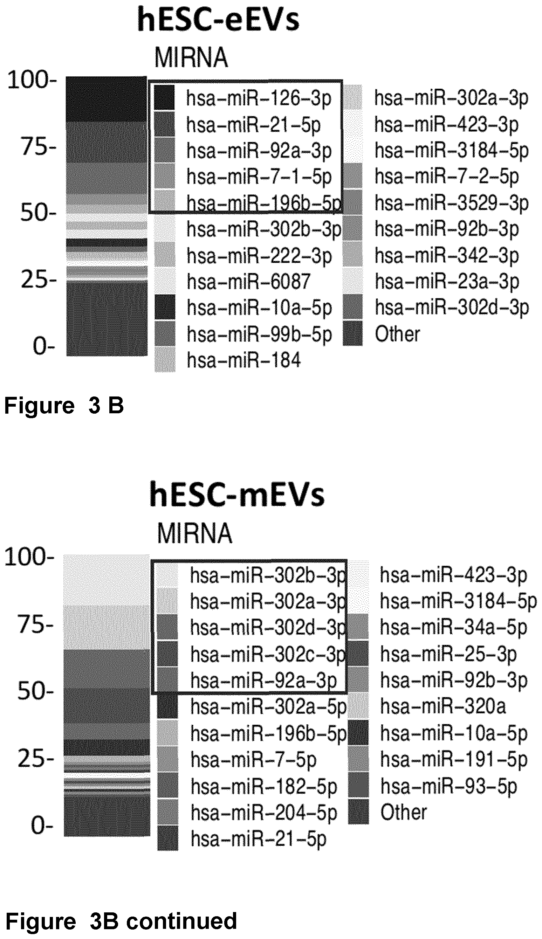

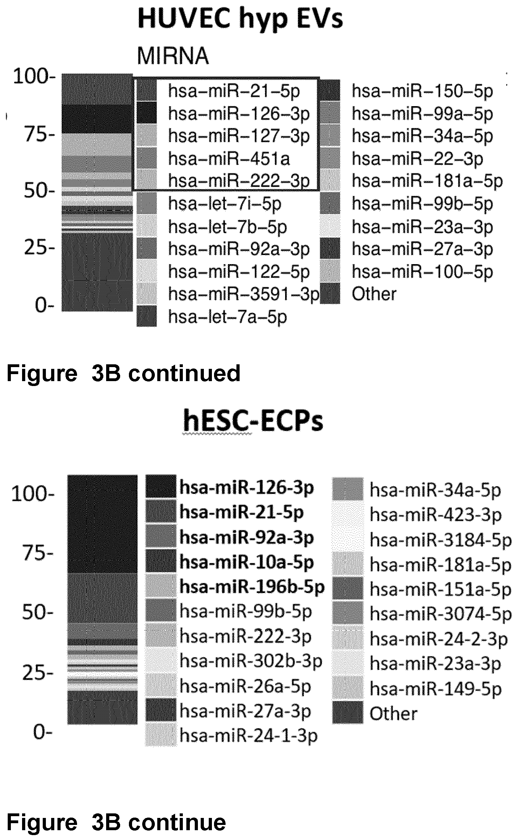

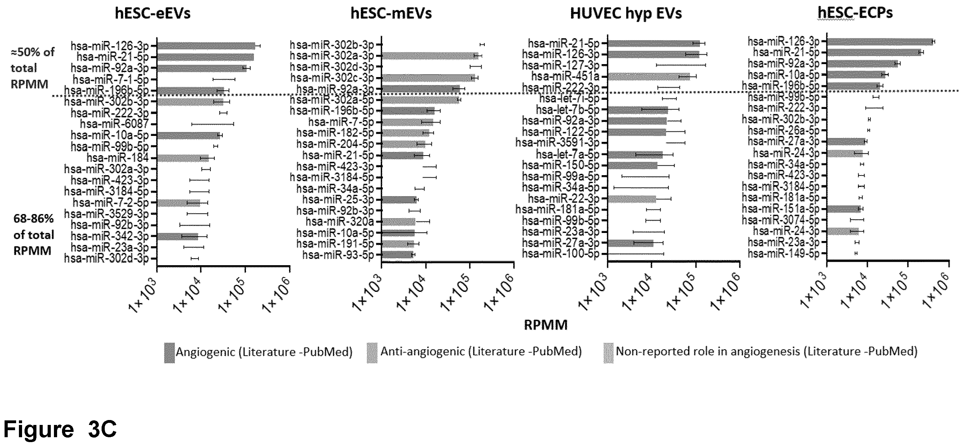

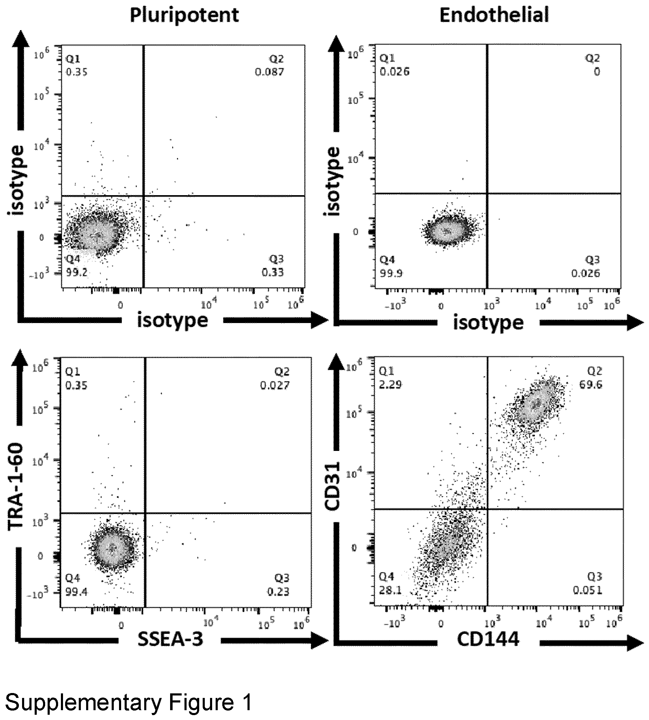

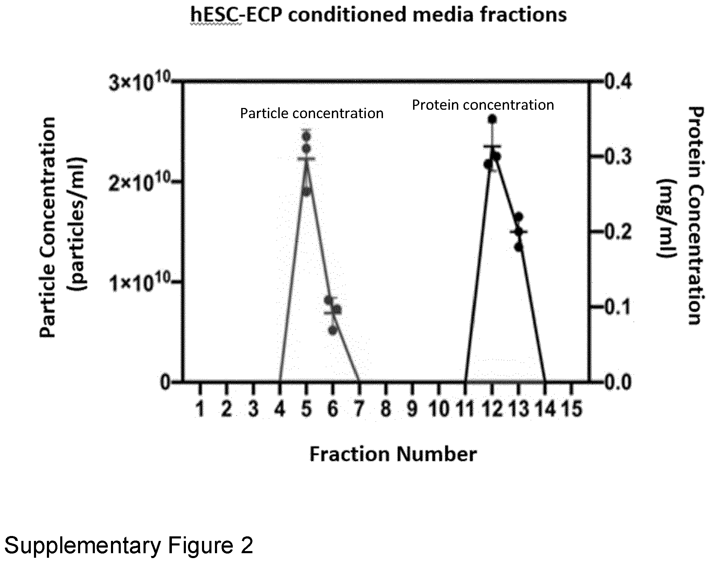

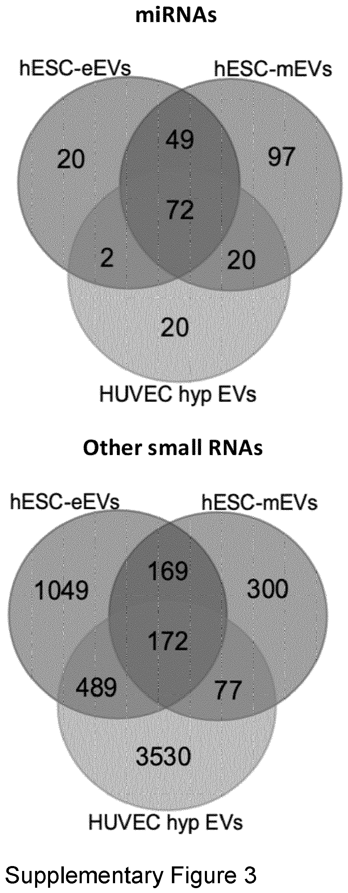

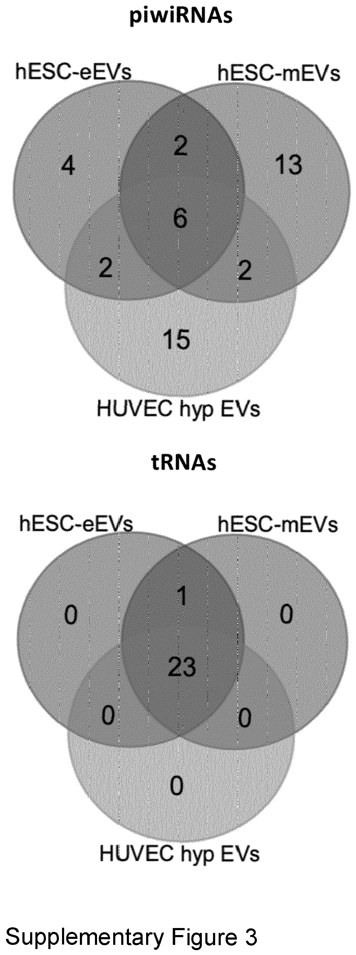

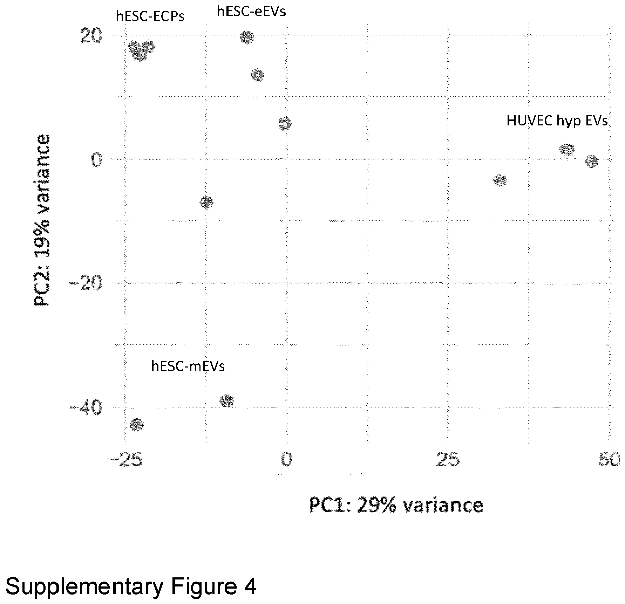

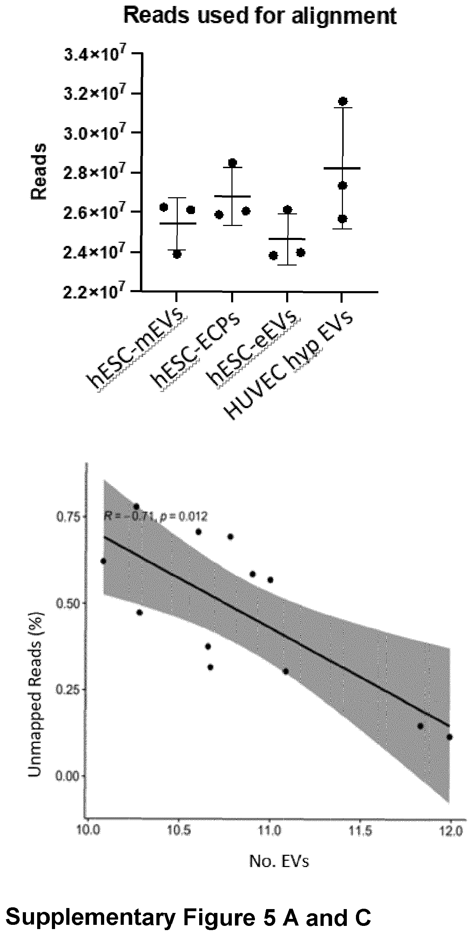

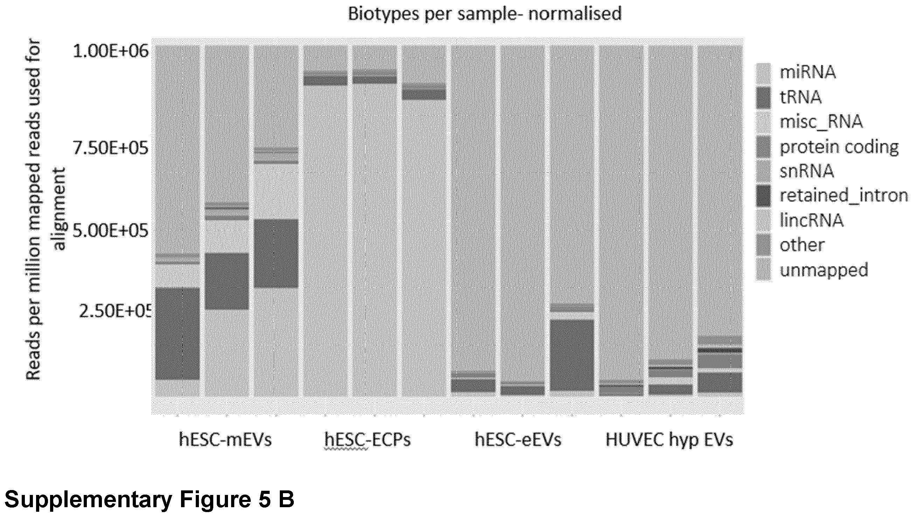

**Figure 3****. hESC-eEVs promote EC wound healing at low concentrations (<100 EVs/cell).** Wound Healing Assay on HCMECs using increasing concentrations of hESC-eEVs (dose range: 1 - 10² EVs/cell) or treated with 10⁵ HUVEC hypoxic EVs/cell (n=4). Cells in fully supplemented media served as positive control (GF+). Cells cultured in basal media and treated with sterile filtered DPBS+P/S (vehicle control) served as negative control (GF-). Graphs (below) represent the percentage of wound closure at 6h, 12h and 24h post-wound induction. Data were analysed using the "MRI Wound Healing" Tool. Black circles correspond to the control samples (GF+ and GF-), purple triangles correspond to HUVEC hypoxic EV treated samples (HUVEC hyp EVs) and green circles correspond to hESC-eEV-treated samples. Statistical significance (indicated with p-values) was determined by one-way ANOVA with Dunnett's multiple comparisons test. Error bars represent the SD. Scale bar=100µm.
**Figure 4****. hESC-eEVs are enriched in angiogenic and the novel miRNAs with a potential role in angiogenesis miR-4496 and miR-4691-5p** A. Graph showing the reads per million mapped of small RNA biotypes in the EV samples and hESC-ECPs (n=3) normalized to the total number of small RNAreads (RPMMtotal). B. Plots representing the percentage of the reads occupied by the top 20 miRNAs of each EV and cellular RNA sample. In all datasets the top 20 miRNAs correspond to 68-86% of the total miRNA reads. The top 5 EV-miRNAs occupy approximately 50% of the total miRNA reads. C. Plots representing the RPMM of the top 20 miRNAs in each group (corresponding to 68-86% of total RPMM) and their role in angiogenesis. Angiogenic miRNAs are highlighted in green and anti-angiogenic miRNAs are highlighted in red (Literature search was conducted on PubMed in July 2022).
**Supplementary** **Figure 1****. Representative flow cytometric analysis of hESC-ECPs.** Cells were stained for endothelial (CD31 and CD144) (right panels), pluripotent markers (TRA-1-60 and SSEA-3) (left panels), and their corresponding isotype controls.
**Supplementary** **Figure 2****. The combination of ultrafiltration with SEC results in good separation of the particle and protein containing fractions.** The graph depicts the particle and protein concentration of hESC-ECP conditioned media fractions resulting after SEC, as determined by NTA and spectrophotometry respectively. Particles are eluted in fractions 5 and 6, while proteins are eluted in fractions 12 and 13.
**Supplementary** **Figure 3****. Small RNA sequencing showed a diverse composition of small RNA classes in EV samples.** Venn diagrams showing unique and common small RNA molecules present in the different EV samples. Molecules with average RPMMtₒₜₐₗ <10 were filtered-out of the analysis.
**Supplementary** **Figure 4****. Principal Component Analysis (PCA) of the total reads of the Small RNA Sequencing datasets.**
**Supplementary Figure 5. Small RNA sequencing read characteristics.** A. RNA input reads used for alignment after filtering out UniVec/rRNA/low quality reads (n=3) B. Stacked bar plots representing read composition per sample. Reads are normalised to the total number of reads (RPMMtotal) C. Correlation between the percentage of unmapped reads and the number of particles used in the small RNA sequencing of the EV samples.

### Materials and methods

### hESC-ECP differentiation

H9 hESC lines were differentiated to endothelial cell products using our previously reported protocol (MacAskill et al., 2018). Human ESC lines were used in accordance with the UK Stem Cell Bank Steering Committee guidelines (Project Approvals SCS11-51 and SCSC17-26). Briefly, hESC were plated on a fibronectin matrix at Day 0 (d0). At d1, lateral mesoderm was induced with GSK3 inhibitor (CHIR99021) (7 µM) and BMP4 (25 ng/mL) added to N2B27/Neurobasal/ DMEM: F12 media. This was followed by endothelial induction at d4 with Forskolin (2 µM) and vascular endothelial growth factor (VEGF) (200ng/mL) in StemPro34 media. In the final step, cells were replated with no matrix and cultured until d8. For EV isolation, media was replaced with EV-free media on d7, and conditioned media was collected after 24 hours. To prepare EV-free media human serum was ultrafiltrated using Amicon-Ultra 15 Centrifugal Filter Units (Merck Millipore) with a cut-off of 100KDa after centrifugation at 3,000xg for 55mins (4°C) and supplemented to the cell culture media.

### hESC-ECP flow cytometry

Day 8 cells were stained using antibodies to determine the proportion of pluripotent (SSEA-4+/TRA-181+) and endothelial (CD31+/CD144+) cells within the population. Flow cytometry was conducted with the BD LDR Fortessa system (Becton) or the Attune NxT system (Thermo Fisher Scientific) and data analysed using the FlowJo software (FlowJo LLC, Ashland, USA).

### EV isolation

To isolate EVs conditioned media was obtained from cells at 70-90% confluency and centrifuged at 3,000xg for 15mins (4°C) to remove cell debris. Supernatants were collected and concentrated to 1ml using Amicon-Ultra 15 Centrifugal Filter Units (Merck Millipore) with a cut-off of 100KDa by centrifugation at 4,000xg for 15mins (4°C). The concentrated samples were loaded onto 15ml sepharose CL-6B (GE Healthcare Bio-Sciences AB) size exclusion chromatography columns. Once the whole sample had entered the column matrix, 0.1µm filtered DPBS (Gibco) supplemented with 1% Penicillin/ Streptomycin (Gibco) was continuously added to ensure complete drainage of the sample. The eluate was collected by gravity in 15 sequential fractions of 1 ml. For each fraction, the amount of protein was determined by spectrophotometry (Absorbance 280nm, Nanodrop, Thermo Fisher Scientific).

### Nanoparticle tracking analysis (NTA)

Particle concentration and size distribution were determined using NanoSight LM 10 instrument (NanoSight Ltd, Amesbury, UK). Samples described above were diluted 10-40 times in 0.1µm filtered DPBS (Gibco) supplemented with 1% Penicillin/ Streptomycin (Gibco) to obtain a concentration of 2*10⁸- 1*10⁹ particles/ml. Camera screen gain was set to 2 and camera level to 16. The settings were kept constant between samples, and each video was analysed to give the mean, mode, median and estimated concentration for each particle size. The analysis was carried out with the NTA software (version 3.3) using 60 seconds of video captures per sample (five replicates per sample). For the video process, the screen gain was set to 14, and the detection threshold to 3.

### Western blot

EV-enriched fractions were concentrated using Amicon-Ultra 15 Centrifugal Filter Units (Merck Millipore) with a cut-off of 100KDa by centrifugation at 4,000xg for 15mins (4°C). For the positive control, cells were lysed in a RIPA lysis buffer (50 mM Tris HCl pH 8, 1% NP-40, 0.2% sodium deoxycholate, 150 mM NaCl, 1% Triton-X-100, 0.1%SDS) supplemented with protease inhibitors (Roche Diagnostics) and centrifuged at 12,000xg for 10 min at 4°C. The supernatants were collected, and the protein concentration was determined with the PierceTM BCA Protein Assay Kit (ThermoFisher Scientific). 5*10¹⁰ particles and 15 mg proteins were separated by NuPAGE^{™} 4-12% Bis-Tris polyacrylamide gels ThermoFisher Scientific) and electrophoretically transferred to nitrocellulose membranes (Invitrogen). The membranes were probed against CD63 (Santa Cruz Biotechnology), CD81 (Santa Cruz Biotechnology) and Calnexin (Abeam).

### Transmission Electron Microscopy (TEM)

Isolated particles were diluted 100 times in 0.1 µm filtered DPBS and mixed 1:1 with 4% methanol-free formaldehyde solution (Thermofisher Scientific). A drop of this solution was placed on a Petri dish, and then a Formvar-coated 200-mesh gold grid (Taab, Aldermaston, UK) floated on top for 20 min. The grid was moved to PBS for two 5 min washes. Then the EVs were re-fixed on the grid using a 1% glutaraldehyde solution for 5 min and again washed twice in PBS. Finally, the grid was transferred to a drop of 0.5% uranyl acetate-2% 25-centipoise methyl cellulose (Sigma Aldrich). Following staining for 5 min, excess fluid was removed, the grid allowed to air dry and was then examined on a JEOL JEM-1400 series 120kV Transmission Electron Microscope. TEM pictures were analysed using "TEM exosome analyzer" (Kotrbova et al., 2019).

### Endothelial Cell Culture

HCMECs and HUVECs were obtained from PromoCell, UK. HCMECs were cultured at 37°C in a humidified atmosphere with 5% CO2 and 95% O in MV2 media (Promocell) supplemented with 5% fetal calf serum (FCS), 5 ng / ml Epidermal Growth Factor (recombinant human), 10ng/ml Basic Fibroblast Growth Factor (recombinant human), 20 ng/ml Insulin-like Growth Factor (Long R3 IGF), 0.5ng/ml VEGF₁₆₅ (recombinant human), 1µg/ml Ascorbic Acid, 0.2 µg/ml Hydrocortisone. HUVECs were cultured in EBM2 media (Promocell) supplemented with 2% fetal calf serum (FCS), 5 ng / ml Epidermal Growth Factor (recombinant human), 10ng/ml Basic Fibroblast Growth Factor (recombinant human), 20 ng/ml Insulin-like Growth Factor (Long R3 IGF), 0.5ng/ml VEGF₁₆₅ (recombinant human), 1µg/ml Ascorbic Acid, 22.5µg/ml heparin, 0.2 µg/ml Hydrocortisone. To isolate EVs from hypoxic HUVECs, when cells reached confluency, media was replaced with EV-free media and transferred to a sealed chamber with 5% CO2, 92% N2 and 3% O2. To prepare EV-free media, FBS was ultrafiltrated using Amicon-Ultra 15 Centrifugal Filter Units (Merck Millipore) with a cut-off of 100KDa after centrifugation at 3,000xg for 55mins (4°C) and supplemented to the cell culture media.

### Tube Formation Assay

The angiogenic effect of hESC-eEVs, hESC-mEVs and HUVEC hypoxic EVs on HCMECs was assessed by the tube formation assay. Cells were stained with 2µM/ml Calcein AM (Invitrogen) after a 30-min incubation at 37°C. Growth Factor-reduced ECM gel (Sigma) was thawed on ice at 4°C overnight, and 10µl was plated per well onto an µ-plate angiogenesis 96-well (Ibidi, UK). After a 30-min gelation period at 37°C, 1.5× 10⁴ HCMECs were seeded onto each well. HCMECs were incubated either with fully supplemented MV2 media (Promocell), or with basal MV2 media (Promocell, UK) with DPBS+P/S, or with basal MV2 media containing increasing concentrations of EVs (1-10⁵ EVs/cell). Bright-field micrographs at 4× were obtained using the EVOS XL Imaging System (Invitrogen). The number of meshes and branches and the total tube length were analysed using the "Angiogenesis analyzer" plug-in (Gilles Carpentier)(Carpentier et al., 2020) on ImageJ software.

### Wound Healing Assay

The ability of hESC-eEVs to promote wound healing *in-vitro* was assessed by the scratch assay. HCMECs were seeded in 6-well plates to create a confluent monolayer. Once they reached 70-90% confluency, the cell monolayer was scratched with a sterile P200 pipette tip and media was replaced to fully supplemented MV2 media (Promocell), or with basal MV2 media (Promocell, UK) with DPBS+P/S, or with basal media containing increasing concentrations of hESC-eEVs (1-10² EVs/cell) or 10⁵ HUVEC hypoxic EVs/cell for 24 h. The wound closure of HCMECs was captured using a bright-field microscope at 0-, 6-, 12- and 24-h post scratch. The percentage of wound coverage, taking the value at 0h as 0%, was calculated using the MRI Wound Healing Tool (Montpellier Resources) on ImageJ software.

### RNA extraction

Total cellular RNA was extracted using a miRNeasy Mini Kit (Qiagen) according to the manufacturer's instructions. The Total exosome RNA and protein isolation kit (Invitrogen) was utilized for the recovery of RNA from the EV samples according to the manufacturer's instructions. RNA quantity and quality were further analysed by the Agilent 2100 Bioanalyzer system (Agilent Technologies, Inc.) using the total RNA Pico Series II chip.

### Small RNA Sequencing

EV isolation by a combination of ultrafiltration with SEC results in high purity EV preparations but with a lower yield than other methods(Théry et al., 2018). To counter the resulting lower input for our libraries we provided the maximum possible input for sequencing each EV library with the minimum total RNA quantity being 1 ng (n=3). Firstly, small RNAs were selected by 15% urea-PAGE gel electrophoresis and gel extraction between 18 to 30 nt. After gel purification, ligation of the adenylated 3' adapter to the small RNA fragments was performed. Reverse transcription (RT) primers with barcodes were used to anneal the 3' adenylated adapter to combine the redundant unligated 3' adenylated adapter. Then, ligation of the 5' adapter and RT reactions were performed. After cDNA first-strand synthesis, the product was amplified by 15 cycles. A second size selection of 103-115 bp fragments from the gel was carried out. This step was conducted to purify the PCR product and remove any nonspecific products. After gel purification, the PCR yield was quantified by Qubit (Invitrogen, Cat No. Q33216). Samples were pooled together to make a single strand DNA circle (ssDNA circle), which gave the final small RNA library. DNA nanoballs (DNBs) were generated with the ssDNA circle by rolling circle replication (RCR) to enlarge the fluorescent signals at the sequencing process. The DNBs were loaded into the patterned nanoarrays, and single-end read of 50 bp were read through on the BGISEQ-500 platform for the following data analysis study (sequencing depth: ≥20 million reads per sample).

### Sequencing read mapping and small RNA annotation

The raw data from the BGI-SEQ500 were in fastq format. We used two mapping and quantification approaches to ensure robust analysis of EV miRNA profiles, the first of which used the exceRpt small RNAseq processing pipeline(Rozowsky et al., 2019). Files were exported to the Genboree Workbench's exceRpt small RNA-seq pipeline (version 4.6.2) for quality control as well as read mapping to the human genome and default small RNA libraries. This allowed for a single-mismatched base down to 18 nucleotides. Reads unsuccessfully clipped (0%), failing quality filter (<1.2%), UniVec contaminants (<0.6%) and rRNA (5-51.4%) were filtered out of the analysis. After these initial filters, the average reads used for alignment for each group ranged from 24.7 to 28.3 million reads (Supplementary Figure 5A). A high percentage of reads could not be aligned to the genome or small RNA libraries and was likely a result of background non-specific amplification in lower input libraries (particularly in hESC-eEVs and HUVEC hypoxic EVs where 30.4-78% reads used for alignment could not be mapped) (Supplementary Figure 5B). We found a negative correlation (rho -0.7, p <0.05, Spearman's rank) between the percentage of unmapped reads and the number of particles used for RNA extraction (as measured by NTA and Agilent Pico Chip Bioanalyzer respectively) suggesting low initial input for particular samples may have led to higher background amplification (Supplementary Figure 5C). We view this low input into sequencing libraries as an acceptable trade-off for the high purity of our EV preparations. Unmapped reads were discarded from all further analyses. Principal component analysis (PCA) of the remaining reads of each sample suggested distinct small RNA profiles (Supplementary Figure 4).

Alignment and quantification of the reads onto the latest release of the reference human genome (GRCh38) was performed with the Shortstack alignment tool(Axtell, 2013; Rozowsky et al., 2019)mapping solely to miRbase. ShortStack offered the ability to provide counts for the same miRNA across multiple genomic loci. Both approaches provided near identical read percentages mapping to miRbase v21. We then compared normalisation of miRNA counts relative to the total counts assigned to miRNAs via Shortstack (RPMM) to the default provided in the exceRpt pipeline which normalises miRNA counts to the total number of reads mapped to all small RNA libraries or the genome (RPMMₜₒₜₐₗ- the exceRpt default). These measures, therefore, quantify individual miRNAs relative to the total pool of miRNAs or the total pool of small RNAs present within EVs respectively. We saw an overall strong positive correlation between RPMM and RPMMₜₒₜₐₗ values (rho 0.76-0.93, p<0.0001, Spearman's rank). However, variability between replicates appeared higher for RPMMₜₒₜₐₗ, especially amongst the highest expressed miRNAs (RPMM>100). Therefore, unless otherwhere specified, RPMM values were used for our analysis. MiRNAs enriched in hESC-eEVs relative to other conditions were determined using ShortStack-derived counts processed by DESeq2(v1.34.0)(Love et al., 2014). We also attenuated overestimates of LFCs due to noise and high variability amongst miRNA counts by using the ashr package(Stephens, 2017) within DESeq2. Comparisons to hESC-mEVs were handled separately to other comparisons due to larger variability between replicates seen via PCA that may unduly influence normalisation for differential expression analysis. Alternative differential expression analysis was also done using the edgeR tool in "classic" approach (Robinson et al., 2010), again separating hESC-mEV comparisons out from others.

### Statistical analysis

All biological replicates using human primary cells correspond to independent experiments from distinct expansions and passage numbers. All graphs (except proportion) are shown as data points including mean ± standard deviation (SD) on biological or technical replicates as detailed in the figure legends. qRT-PCR data in graphs are shown as relative expressions as described by Livak and Schmittgen(Livak & Schmittgen, 2001). Statistical analysis was performed using GraphPad Prism 9.0.0 (GraphPad Software, La Jolla, CA, USA). Tests used to assess significance are detailed in each Figure legend and precise p-values of significant changes are indicated on the graph. A value of p < 0.05 was the level of nominal significance. For in-vitro experiments, as each experimental data set is an average of a large number of cultured cells, we assumed the data was normally distributed based on the central limit theorem. One-way ANOVA with Dunnett's multiple comparisons test was used to determine significant differences between the samples in our *in-vitro* experiments (>2 groups).

### Results

### 1. EVs isolated from hESC-ECP conditioned media express EV markers and show phenotypic characteristics of exosomes

To isolate hESC-eEVs, pluripotent hESCs underwent an established 8-day differentiation protocol via a mesodermal stage (Figure 1A) as previously described. We confirmed that at Day 8, two-thirds (65.7%) of the cells expressed the EC cell surface markers CD31 and CD144, with fewer than 0.05% co-expressing pluripotent markers SSEA-3/TRA-1-60 as shown by flow cytometry (Supplementary Figure 1). A combination of ultrafiltration and size exclusion chromatography (SEC) was used to isolate EVs from the hESC-ECP conditioned media (Figure 1B). To select fractions enriched for EVs and devoid of protein contamination, the protein and EV concentration of all fractions was measured by spectrophotometry and Nanoparticle Tracking Analysis (NTA) respectively. Fractions 5 and 6 containing considerably higher numbers of particles and low protein concentrations (Supplementary Figure 2) were pooled and used for further analysis to confirm isolation of EVs.

Enrichment of EVs in the isolated fractions was assessed by characterisation of the EV size distribution, surface markers and morphology. NTA showed that the size of particles in the pooled fractions 5 and 6 ranged from 30 to 200 nm with a mean value of 84 ± 7.3 (Figure 1C). Western blot analysis confirmed that the particles were positive for the EV markers CD63 and CD81 while negative for the endoplasmic reticulum marker Calnexin (Figure 1D). By transmission electron microscopy (TEM), structures of the characteristic size and shape of EVs were identified. Quantification of the TEM images showed that the average EV size was 100.42 ±38.36 and the roundness ratio was 0.96±0.06 (Figure 1E). Thus, EV preparations from hESC-ECP conditioned media through a combination of ultrafiltration and SEC resulted in the isolation of particles with the characteristic size, morphology, and markers (CD63 and CD81) of EVs whilst also being devoid of protein contamination. These high purity EV preparations are henceforth referred to as hESC-eEVs.

### 2. hESC-eEVs are internalised by ECs and promote tube formation and wound closure at low concentrations (<100 EVs/cell)

Since increased EC tube formation and wound closure are considered hallmarks of angiogenesis(S. Guo et al., 2014) and previous reports have demonstrated that the effect of EC-derived EVs in angiogenesis depends on the dose used(Lacroix et al., 2007), to assess the angiogenic potency of hESC-eEVs we first performed tube formation and wound closure assays using increasing concentrations of hESC-eEVs isolated from 4 independent differentiations (i.e., n=4). HCMECs cultured in fully supplemented media were used as positive controls of angiogenic/migratory cells, and cells cultured in media without growth factors and treated with equal volumes of sterile 0.1 µm filtered PBS (EV-suspension solution) were used as negative controls. Since hypoxia induces the release of angiogenic EVs (Bister et al., 2020), we also treated HCMECs with EVs obtained from hypoxic Human Umbilical Vein ECs (HUVECs), as an EC-derived EV control. Performing dose-response experiments using HUVEC hypoxic EVs we demonstrated that these EVs were ineffective at inducing EC tube formation at low doses (<100EVs/cell) and showed the highest effect at high doses (10⁵ EVs/cell) (Figure 2A). In contrast to this, treatment with approximately 1 hESC-eEV per cell resulted in significantly increased HCMEC tube formation ability compared to the negative control (p<0.0001) (Figure 2B). Thus, a considerably lower dose of hESC-eEVs (1EV/cell) was required to promote HCMEC tube formation at levels similar to the HUVEC hypoxic EVs (10⁵ EVs/cell). We also showed that as opposed to HUVEC hypoxic EVs, treatment with a high hESC-eEV concentration (≥100 EVs/cell) failed to replicate this effect. Staining with Calcein AM confirmed cell viability in all conditions in the tube formation assay (Figure 2C).

To understand whether the effect on angiogenesis is specific for EVs from the endothelial stage of the hESC-ECP differentiation system, we investigated the effect of EVs derived from the mesodermal stage of the differentiation protocol (hESC-mEVs) on HCMEC tube formation ability (dose range: 1-10⁵ EVs/cell). Cells cultured in fully supplemented media, or in basal media but treated with 1 hESC-eEV/cell were used as positive controls, while cells cultured in basal media and treated with PBS were used as negative controls. Our results showed that in contrast to treatment with 1 hESC-eEV/cell, treatment with hESC-mEVs did not induce tube formation in any dose tested (Figure 2D). Thus, the ability to promote angiogenesis is specific for EVs derived from the endothelial stage of the ESC-ECP differentiation system (hESC-eEVs) and is absent in EVs from the mesodermal stage (hESC-mEVs). To confirm that the angiogenic effect observed post-hESC-eEV treatment is specific for the EVs and not for other soluble, non-EV-associated secreted factors, we subjected complete medium (not exposed to cells) to the EV purification process and collected samples at different stages of the purification (Figure 2E). These samples were tested for their ability to induce HCMEC tube formation (Figure 2F). Cells cultured in fully supplemented media, or in basal media but treated with 1 hESC-eEV/cell were used as positive controls, while cells cultured in basal media and treated with PBS were used as negative controls. Since the cell culture media contains a high concentration of human recombinant VEGFA₁₆₅ and VEGFA is a key driver of angiogenesis (Apte et al., 2019), cells treated with human recombinant VEGFA₁₆₅ were also used as positive controls for this experiment. Our results showed that treatment with complete medium subjected to the EV purification process does not improve HCMEC tube formation ability, while treatment with EVs isolated from conditioned media (1 hESC-eEV/cell) promoted HCMEC tube formation. Therefore, it is unlikely that soluble, non-EV-associated secreted factors contribute to the angiogenic effect observed post-hESC-eEV treatment.

Since we observed that hESC-eEVs induce HCMEC tube formation at low concentrations, to assess the effect of hESC-eEVs on EC wound healing, we performed scratch assays using increasing concentrations of hESC-eEVs with the maximum dose being 100 hESC-eEVs/cell (Figure 3). Our results demonstrated that 6-24h after the induction of the wound, HCMECs treated with low hESC-eEV doses (<100 EVs/cell) migrated significantly faster than cells cultured in basal media and treated with PBS. Cells treated with a high hESC-eEV concentration (100 EVs/cell) or with HUVEC hypoxic EVs did not show significantly improved migratory ability compared to the negative control. Collectively, these results indicate the potency of the hESC-eEVs to stimulate angiogenesis at low concentrations (<100EVs/cell). It also appears that this angiogenic action is lost at higher hESC-eEV concentrations, suggesting dose-specific effects. Moreover, our data demonstrate that the ability to induce angiogenesis is specific for EVs derived from the endothelial and not the mesodermal stage of the hESC-ECP differentiation.

### 3. hESC-eEVs are enriched in angiogenic and other miRNAs with a potential role in angiogenesis

Since the effect of EVs has been largely attributed to their small RNA cargo (O'Brien et al., 2020), we aimed to identify RNA molecules responsible for the angiogenic activity of hESC-eEVs. Small RNA was extracted from EVs from the following samples: hESC-eEVs, hESC-mEVs and HUVEC hypoxic EVs (n=3 independent stem cell differentiations) and we also obtained RNA from hESC-ECPs to identify EV-enriched miRNAs. RNA size profiling showed the enrichment of small RNAs (25-200nt) in EVs while ribosomal and longer RNAs were absent. Cellular RNA sample profiling showed two distinct ribosomal peaks corresponding to 18S and 28S for eukaryotic RNA. Small RNA sequencing was performed on these RNA samples. The majority of the cellular RNA sample reads mapped to sense miRNAs, while EV RNA samples showed a more diverse composition of small RNA classes, mapping mostly to tRNAs, miRNAs, miscellaneous RNAs (misc_RNA) and protein-coding molecules (Figure 4A). To understand if EVs obtained from different conditions have distinct RNA molecules, we compared the list of unique miRNAs, piRNAs, tRNAs and other RNAs between EV types (Supplementary Figure 3). TRNAs and piwiRNAs were less variable, while miRNAs and other small RNAs (including miscellaneous RNA, snRNA, yRNA, retained introns, protein coding molecules, lincRNA etc) were the most heterogeneous populations among the EV samples. Overall, the different EV samples shared a total of 72 miRNAs and 172 other small RNAs. In this study, we focused on miRNAs for further investigation since EV-miRNAs have a well-established role in the regulation of angiogenesis (Kesidou et al., 2020) in cardiovascular diseases (CVDs).

We next assessed for the presence of EC-enriched miRNAs (miR-126-5p, miR-222-3p, miR-99b-5p, miR-22a-3p)(de Rie et al., 2017) in our datasets and saw high abundance in the hESC-eEVs and HUVEC hypoxic EVs and not in hESC-mEVs. In line with previous findings on cellular RNA composition reporting that for any human cell type the most abundant 3-5 miRNAs occupy more than 50% of the total miRNA pool (de Rie et al., 2017), we found that the top 3 miRNAs in hESC-ECPs corresponded to more than 50% of the total miRNA reads. A similar profile was observed in the EV samples were the top 5 EV miRNAs corresponded to approximately 50% of the total miRNA reads (Figure 4B). A literature search of the 20 most abundant miRNAs of each sample (that corresponded to 68-86% of the total reads) showed the presence of several miRNAs with non-reported roles in angiogenesis in hESC-eEVs. The top 5 miRNAs in hESC-eEVs contain molecules extensively studied for their roles in driving angiogenesis, while the top 5 miRNAs in hESC-mEVs have an anti-angiogenic action(Cao et al., 2019; Fish et al., 2008; B. Guo et al., 2017; Jakob et al., 2012; Jansen et al., 2013; Li et al., 2016; Liu et al., 2019; Qiao et al., 2019; Qu et al., 2019; Q.-Z. Wang et al., 2021; S. Wang et al., 2008) (Figure 4C). Taken together, our data suggest that hESC-eEVs are enriched in angiogenic miRNAs as well as miRNAs with non-reported roles in angiogenesis.

### Discussion

Since our previous data suggested that the improvements observed post-hESC-ECPs transplantation may be due to paracrine mechanisms(MacAskill et al., 2018), here we aimed to investigate the effect of hESC-eEVs in angiogenesis. The present study provides several novel findings in the EV field. We developed a reproducible protocol for the isolation of pure and biologically active EVs from hESC-ECP cultures and demonstrated that compared to other EC-derived EVs, hESC-eEVs induce angiogenesis at low concentrations. We also showed that hESC-eEVs are enriched in angiogenic and other miRNAs with non-reported roles in angiogenesis.

In the present study, EVs were isolated by a combination of ultrafiltration with SEC, which leads to the isolation of highly pure EV populations (Théry et al., 2018). Although the EV yield is lower compared to other approaches (Théry et al., 2018), the combination of ultrafiltration with SEC has been proposed as a promising strategy to isolate biologically active EVs(Benedikter et al., 2017; Mol et al., 2017). Using this approach, we confirmed the isolation of EVs with intact ultrastructure, positive for the EV markers CD63 and CD81 and negative for the cellular contamination marker, calnexin (Kozlov & Gehring, 2020) and devoid of protein contamination. To investigate the effect of hESC-eEVs in angiogenesis, we performed tube formation and scratch assays on ECs. We also compared the effects of our EVs to EVs isolated from the mesodermal stage of the hESC-ECP differentiation (hESC-mEVs) and to mature ECs. As a mature EC population, we selected HUVEC hypoxic EVs since hypoxia induces the release of angiogenic EVs (Bister et al., 2020). Our results showed that hESC-eEVs at low concentrations (<100 EV/cell) significantly increased EC angiogenic ability, while a much higher dose of HUVEC hypoxic EVs was required to observe similar effects (10⁵ EV/cell). We also showed that treatment with a high hESC-eEV concentration (≥100 EVs/cell) does not affect EC angiogenic ability. This may suggest the requirement of a precise dosage for the hESC-eEV-mediated angiogenic effects. Similar trends have been reported in the past in other angiogenesis studies using EC-derived EVs in increasing doses (Lacroix et al., 2007; Ou et al., 2011). However, the use of different methodologies for EV isolation and characterisation makes the comparison between individual EV studies challenging. Another important factor to consider is that as a particle quantification technique, NTA has several disadvantages, including that it may lead to underestimation of the particle concentration (Bachurski et al., 2019). Thus, the precise estimation of EV doses used in individual experiments remains a key hurdle in the field. In contrast to EC-derived EVs, we showed that treatment with EVs from the mesodermal stage of the hESC-ECP differentiation (hESC-mEVs) did not affect EC angiogenic ability, which suggests that the ability to induce angiogenesis is specific to EVs isolated from the more mature stage of the hESC-ECP differentiation. Overall, the requirement of a very low dose of the hESC-eEVs to yield the improved effects may be promising for the clinical application of hESC-eEVs for therapeutic angiogenesis.

One of the most useful tools to study the EV miRNA cargo is small RNA sequencing since it presumably provides an unbiased view of EV-miRNA content. To identify key miRNA candidates that may drive the angiogenic potential of hESC-eEVs we performed small RNA sequencing and compared the small RNA cargo of the hESC-eEVs to the one of hESC-mEVs, HUVEC hypoxic EVs and the EV-donor cells. EV small RNA sequencing was performed by DNBSEQ^{™} NGS technology platform. A possible limitation in our approach was the low-RNA input in the EV samples which was mainly due to 2 reasons: the hESC-eEV-source which comes from a differentiation system, and the low yield in the EV-isolation by a combination of ultrafiltration and SEC(Théry et al., 2018), which we found to be correlated to the high percentage of unmapped reads in our libraries. The high purity-low depth approach of our EV isolation and sequencing may miss out on full detection of all EV miRNAs, however, we provide sufficient coverage to profile the most highly abundant miRNAs which are those most likely to influence angiogenesis at the low concentrations required for hESC-eEVs. Our results showed that the majority of the EV RNA samples mapped to tRNAs, miRNAs, misc_RNA and protein-coding molecules, while most cellular RNA sample reads mapped to sense miRNAs. We noted miRNAs and other small RNAs (including miscellaneous RNA, snRNA, yRNA, retained introns, protein-coding molecules, IincRNA etc) being the most heterogeneous populations among the different EV samples but focused on miRNAs for the rest of our analysis since the role of miRNAs in angiogenesis is better understood(Kesidou et al., 2020).

Our small RNA sequencing analysis revealed for the first time that in line with previous findings on cellular RNA composition reporting that for any human cell type the top 3-5 miRNAs occupy more than 50% of the total miRNA pool(de Rie et al., 2017) the top 5 EV miRNAs also correspond to approximately 50% of the total miRNA reads. We showed that the most abundant miRNAs in hESC-eEVs were molecules with well-established roles in the induction of angiogenesis, while the most abundant miRNAs in hESC-mEVs have an anti-angiogenic action which indicates why a low dose of hESC-eEVs is required to induce angiogenesis and could possibly explain why the hESC-mEVs were ineffective at inducing angiogenesis in our *in-vitro* experiments. Our results also suggest that the most abundant miRNAs in HUVEC hypoxic EVs were molecules with diverse roles in driving angiogenesis, which may explain why a higher dose of HUVEC hypoxic EVs is required to observe similar effects on angiogenesis to hESC-eEVs. Differential expression analysis was performed to assess whether the differences in the EV angiogenic action can be explained by their miRNA cargo. Our results showed that several miRNAs were differentially expressed between the angiogenic hESC-eEVs and the non-angiogenic hESC-mEVs. The most enriched miRNA in hESC-eEVs compared to the hESC-mEVs was the EC specific (de Rie et al., 2017) miRNA miR-126-3p whose role in driving angiogenesis has been extensively studied (Qu et al., 2019).

Overall, this study is the first to investigate the effect of hESC-eEVs in angiogenesis, to provide a comprehensive insight into the miRNA content of hESC-eEVs, and to compare the miRNA cargo of hESC-eEVs to hESC-mEVs, mature EC-EVs. Our data highlight a number of elements that will contribute to both the EV and the CVD field: (1) compared to other EC-derived EVs, hESC-eEVs induce angiogenesis at low concentrations, while EVs from more immature stages of the hESC-ECP differentiation protocol are not angiogenic, (2) hESC-eEVs are enriched in angiogenic, while hESC-mEVs in anti-angiogenic miRNAs and the most abundant miRNAs in HUVEC hypoxic EVs are molecules with versatile roles in driving angiogenesis.

### References

Ana-Mishel Spiroski, Ian R. McCracken, Adrian Thomson, Marlene Magalhaes-Pinto, Mukesh K. Lalwani, Kathryn J. Newton, Eileen Miller, Cecile Benezech, Patrick Hadoke, Mairi Brittan, Joanne C. Mountford, Abdelaziz Beqqali, Gillian A. Gray, & Andrew H. Baker. (2022). Human embryonic stem cell-derived endothelial cell product injection attenuates cardiac remodeling in myocardial infarction. Front. Cardiovasc. Med.
Apte, R. S., Chen, D. S., & Ferrara, N. (2019). VEGF in Signaling and Disease: Beyond Discovery and Development. Cell, 176(6), 1248-1264. https://doi.org/10.1016/J.CELL.2019.01.021
Axtell, M. J. (2013). ShortStack: Comprehensive annotation and quantification of small RNA genes. RNA, 19(6), 740. https://doi.org/10.1261/RNA.035279.112
Bachurski, D., Schuldner, M., Nguyen, P.-H., Malz, A., Reiners, K. S., Grenzi, P. C., Babatz, F., Schauss, A. C., Hansen, H. P., Hallek, M., & Pogge von Strandmann, E. (2019). Extracellular vesicle measurements with nanoparticle tracking analysis - An accuracy and repeatability comparison between NanoSight NS300 and ZetaView. Journal of Extracellular Vesicles, 8(1), 1596016. https://doi.org/10.1080/20013078.2019.1596016
Benedikter, B. J., Bouwman, F. G., Vajen, T., Heinzmann, A. C. A., Grauls, G., Mariman, E. C., Wouters, E. F. M., Savelkoul, P. H., Lopez-Iglesias, C., Koenen, R. R., Rohde, G. G. U., & Stassen, F. R. M. (2017). Ultrafiltration combined with size exclusion chromatography efficiently isolates extracellular vesicles from cell culture media for compositional and functional studies. Scientific Reports 2017 7:1, 7(1), 1-13. https://doi.org/10.1038/s41598-017-15717-7
Bister, N., Pistono, C., Huremagic, B., Jolkkonen, J., Giugno, R., & Malm, T. (2020). Hypoxia and extracellular vesicles: A review on methods, vesicular cargo and functions. Journal of Extracellular Vesicles, 10(1). https://doi.org/10.1002/jev2.12002
Cao, J., Li, L., Han, X., Cheng, H., Chen, W., Qi, K., Chen, C., Wu, Q., Niu, M., Zeng, L., & Xu, K. (2019). miR-302 cluster inhibits angiogenesis and growth of K562 leukemia cells by targeting VEGFA. OncoTargets and Therapy, 12, 433-441. https://doi.org/10.2147/OTT.S190146
Carpentier, G., Berndt, S., Ferratge, S., Rasband, W., Cuendet, M., Uzan, G., & Albanese, P. (2020). Angiogenesis Analyzer for ImageJ - A comparative morphometric analysis of "Endothelial Tube Formation Assay" and "Fibrin Bead Assay." Scientific Reports 2020 10:1, 10(1), 1-13. https://doi.org/10.1038/s41598-020-67289-8
Chekanov, V., Akhtar, M., Tchekanov, G., Dangas, G., Shehzad, M. Z., Tio, F., Adamian, M., Colombo, A., Poubin, G., Leon, M. B., Moses, J. W., & Kipshidze, N. N. (2003). Transplantation of autologous endothelial cells induces angiogenesis. Pacing and Clinical Electrophysiology: PACE, 26(1P2), 496-499. https://doi.org/10.1046/J.1460-9592.2003.00080.X
Dard-Dascot, C., Naquin, D., d'Aubenton-Carafa, Y., Alix, K., Thermes, C., & van Dijk, E. (2018). Systematic comparison of small RNA library preparation protocols for next-generation sequencing. BMC Genomics, 19(1), 1-16. https://doi.org/10.1186/512864-018-4491-6/FIGURES/5
de Rie, D., Abugessaisa, I., Alam, T., Arner, E., Arner, P., Ashoor, H., Åström, G., Babina, M., Bertin, N., Burroughs, A. M., Carlisle, A. J., Daub, C. O., Detmar, M., Deviatiiarov, R., Fort, A., Gebhard, C., Goldowitz, D., Guhl, S., Ha, T. J., ... de Hoon, M. J. L. (2017). An integrated expression atlas of miRNAs and their promoters in human and mouse. Nature Biotechnology 2017 35:9, 35(9), 872-878. https://doi.org/10.1038/nbt.3947
Fish, J. E., Santoro, M. M., Morton, S. U., Yu, S., Yeh, R. F., Wythe, J. D., Ivey, K. N., Bruneau, B. G., Stainier, D. Y. R., & Srivastava, D. (2008). miR-126 Regulates Angiogenic Signaling and Vascular Integrity. Developmental Cell, 15(2), 272-284. https://doi.org/10.1016/j.devce1.2008.07.008
Gnecchi, M., Zhang, Z., Ni, A., & Dzau, V. J. (2008). Paracrine mechanisms in adult stem cell signaling and therapy. Circulation Research, 103(11), 1204-1219. https://doi.org/10.1161/CIRCRESAHA.108.176826
Guo, B., Zhao, Z., Wang, Z., Li, Q., Wang, X., Wang, W., Song, T., & Huang, C. (2017). MicroRNA-302b-3p Suppresses Cell Proliferation Through AKT Pathway by Targeting IGF-1R in Human Gastric Cancer. Cellular Physiology and Biochemistry, 42(4), 1701-1711. https://doi.org/10.1159/000479419
Guo, S., Lok, J., Liu, Y., Hayakawa, K., Leung, W., Xing, C., Ji, X., & Lo, E. H. (2014). Assays to examine endothelial cell migration, tube formation, and gene expression profiles. Methods in Molecular Biology (Clifton, N.J.), 1135, 393-402. https://doi.org/10.1007/978-1-4939-0320-7_32
Hill, A. F., Pegtel, D. M., Lambertz, U., Leonardi, T., O'Driscoll, L., Pluchino, S., Ter-Ovanesyan, D., & Nolte-'t Hoen, E. N. M. (2013). ISEV position paper: extracellular vesicle RNA analysis and bioinformatics. Journal of Extracellular Vesicles, 2(1), 22859. https://doi.org/10.3402/JEV.V210.22859
Irvin, M. W., Zijlstra, A., Wikswo, J. P., & Pozzi, A. (2014). Techniques and assays for the study of angiogenesis. Experimental Biology and Medicine (Maywood, N.J.), 239(11), 1476. https://doi.org/10.1177/1535370214529386
Jakob, P., Doerries, C., Briand, S., Mocharla, P., Kränkel, N., Besler, C., Mueller, M., Manes, C., Templin, C., Baltes, C., Rudin, M., Adams, H., Wolfrum, M., Noll, G., Ruschitzka, F., Lüscher, T. F., & Landmesser, U. (2012). Loss of angiomir-126 and 130a in angiogenic early outgrowth cells from patients with chronic heart failure: Role for impaired in vivo neovascularization and cardiac repair capacity. Circulation, 126(25), 2962-2975. https://doi.org/10.1161/CIRCULATIONAHA.112.093906
Jansen, F., Yang, X., Hoelscher, M., Cattelan, A., Schmitz, T., Proebsting, S., Wenzel, D., Vosen, S., Franklin, B. S., Fleischmann, B. K., Nickenig, G., & Werner, N. (2013). Endothelial microparticle-mediated transfer of microRNA-126 promotes vascular endothelial cell repair via spred1 and is abrogated in glucose-damaged endothelial microparticles. Circulation, 128(18), 2026-2038. https://doi.org/10.1161/CIRCULATIONAHA.113.001720/FORMAT/EPUB
Kesidou, D., da Costa Martins, P. A., de Windt, L. J., Brittan, M., Beqqali, A., & Baker, A. H. (2020). Extracellular Vesicle miRNAs in the Promotion of Cardiac Neovascularisation. Frontiers in Physiology, 11. https://doi.org/10.3389/FPHYS.2020.579892
Koga, H., Sugiyama, S., Kugiyama, K., Watanabe, K., Fukushima, H., Tanaka, T., Sakamoto, T., Yoshimura, M., Jinnouchi, H., & Ogawa, H. (2005). Elevated Levels of VE-Cadherin-Positive Endothelial Microparticles in Patients With Type 2 Diabetes Mellitus and Coronary Artery Disease. Journal of the American College of Cardiology, 45(10), 1622-1630. https://doi.org/10.1016/j.jacc.2005.02.047
Kotrbová, A., Štěpka, K., Maška, M., Pálenik, J. J., Ilkovics, L., Klemová, D., Kravec, M., Hubatka, F., Dave, Z., Hampl, A., Bryja, V., Matula, P., & Pospíchalová, V. (2019). TEM ExosomeAnalyzer: a computer-assisted software tool for quantitative evaluation of extracellular vesicles in transmission electron microscopy images. *Journal of Extracellular Vesicles, 8*(1)*.* https://doi.org/10.1080/20013078.2018.1560808
Kozlov, G., & Gehring, K. (2020). Calnexin cycle - structural features of the ER chaperone system. The FEBS Journal, 287(20), 4322-4340. https://doi.org/10.1111/FEBS.15330
Lacroix, R., Sabatier, F., Mialhe, A., Basire, A., Pannell, R., Borghi, H., Robert, S., Lamy, E., Plawinski, L., Camoin-Jau, L., Gurewich, V., Angles-Cano, E., & Dignat-George, F. (2007). Activation of plasminogen into plasmin at the surface of endothelial microparticles: A mechanism that modulates angiogenic properties of endothelial progenitor cells in vitro. Blood, 110(7), 2432-2439. https://doi.org/10.1182/blood-2007-02-069997
Li, Y. Z., Wen, L., Wei, X., Wang, Q. R., Xu, L. W., Zhang, H. M., & Liu, W. C. (2016). Inhibition of miR-7 promotes angiogenesis in human umbilical vein endothelial cells by upregulating VEGF via KLF4. Oncology Reports, 36(3), 1569-1575. https://doi.org/10.3892/OR.2016.4912
Liu, Y., Li, Q., Hosen, M. R., Zietzer, A., Flender, A., Levermann, P., Schmitz, T., Frühwald, D., Goody, P., Nickenig, G., Werner, N., & Jansen, F. (2019). Atherosclerotic conditions promote the packaging of functional MicroRNA-92a-3p into endothelial microvesicles. Circulation Research, 124(4), 575-587. https://doi.org/10.1161/CIRCRESAHA.118.314010
Livak, K. J., & Schmittgen, T. D. (2001). Analysis of relative gene expression data using real-time quantitative PCR and the 2(-Delta Delta C(T)) Method. Methods (Son Diego, Calif.), 25(4), 402-408. https://doi.org/10.1006/METH.2001.1262
Love, M. I., Huber, W., & Anders, S. (2014). Moderated estimation of fold change and dispersion for RNA-seq data with DESeq2. Genome Biology, 15(12), 1-21. https://doi.org/10.1186/S13059-014-0550-8/FIGURES/9
MacAskill, M. G., Saif, J., Condie, A., Jansen, M. A., MacGillivray, T. J., Tavares, A. A. S., Fleisinger, L., Spencer, H. L., Besnier, M., Martin, E., Biglino, G., Newby, D. E., Hadoke, P. W. F., Mountford, J. C., Emanueli, C., & Baker, A. H. (2018). Robust Revascularization in Models of Limb ischemia Using a Clinically Translatable Human Stem Cell-Derived Endothelial Cell Product. Molecular Therapy, 26(7), 1669-1684. https://doi.org/10.1016/j.ymthe.2018.03.017
Martínez-González, E., Brochado-Kith, Ó., Gómez-Sanz, A., Martín-Carbonero, L., Jimenez-Sousa, M. Á., Martínez-Román, P., Resino, S., Briz, V., & Fernández-Rodríguez, A. (2020). Comparison of methods and characterization of small RNAs from plasma extracellular vesicles of HIV/HCV coinfected patients. Scientific Reports 2020 10:1, 10(1), 1-13. https://doi.org/10.1038/s41598-020-67935-1
McCracken, I. R., Taylor, R. S., Kok, F. O., de la Cuesta, F., Dobie, R., Henderson, B. E. P., Mountford, J. C., Caudrillier, A., Henderson, N. C., Ponting, C. P., & Baker, A. H. (2019). Transcriptional dynamics of pluripotent stem cell-derived endothelial cell differentiation revealed by single-cell RNA sequencing. European Heart Journal, https://doi.org/10.1093/eurheartj/ehz351
Mol, E. A., Goumans, M. J., Doevendans, P. A., Sluijter, J. P. G., & Vader, P. (2017). Higher functionality of extracellular vesicles isolated using size-exclusion chromatography compared to ultracentrifugation. Nanomedicine: Nanotechnology, Biology and Medicine, 13(6), 2061-2065. https://doi.org/10.1016/J.NANO.2017.03.011
O'Brien, K., Breyne, K., Ughetto, S., Laurent, L. C., & Breakefield, X. O. (2020). RNA delivery by extracellular vesicles in mammalian cells and its applications. Nature Reviews Molecular Cell Biology 2020 21:10, 21(10), 585-606. https://doi.org/10.1038/s41580-020-0251-y
Ou, Z.-J., Chang, F.-J., Luo, D., Liao, X.-L., Wang, Z.-P., Zhang, X., Xu, Y.-Q., & Ou, J.-S. (2011). Endothelium-derived microparticles inhibit angiogenesis in the heart and enhance the inhibitory effects of hypercholesterolemia on angiogenesis. Am J Physiol Endocrinol Metab, 300, 661-668. https://doi.org/10.1152/ajpendo.00611.2010.-Therapeutic
Qiao, L., Hu, S., Liu, S., Zhang, H., Ma, H., Huang, K., Li, Z., Su, T., Vandergriff, A., Tang, J., Allen, T., Dinh, P.-U., Cores, J., Yin, Q., Li, Y., & Cheng, K. (2019). microRNA-21-5p dysregulation in exosomes derived from heart failure patients impairs regenerative potential. The Journal of Clinical Investigation, 129(6), 2237-2250. https://doi.org/10.1172/JCI123135
Qu, M., Pan, J., Wang, L., Zhou, P., Song, Y., Wang, S., Jiang, L., Geng, J., Zhang, Z., Wang, Y., Tang, Y., & Yang, G.-Y. (2019). MicroRNA-126 Regulates Angiogenesis and Neurogenesis in a Mouse Model of Focal Cerebral Ischemia. Molecular Therapy Nucleic Acids. https://doi.org/10.1016/j.omtn.2019.02.002
Ridger, V. C., Boulanger, C. M., Angelillo-Scherrer, A., Badimon, L., Blanc-Brude, O., Bochaton-Piallat, M. L., Boilard, E., Buzas, E. I., Caporali, A., Dignat-George, F., Evans, P. C., Lacroix, R., Lutgens, E., Ketelhuth, D. F. J., Nieuwland, R., Toti, F., Tuñon, J., Weber, C., Hoefer, I. E., ... Harrison, P. (2017). Microvesicles in vascular homeostasis and diseases position paper of the european society of cardiology (ESC) working group on atherosclerosis and vascular biology. Thrombosis and Haemostasis, 117(7), 1296-1316. https://doi.org/10.1160/TH16-12-0943
Robinson, M. D., McCarthy, D. J., & Smyth, G. K. (2010). edgeR: a Bioconductor package for differential expression analysis of digital gene expression data. Bioinformatics, 26(1), 139-140. https://doi.org/10.1093/BIOINFORMATICS/BTP616
Pozowsky, J., Kitchen, R. R., Park, J. J., Subramanian, S. L., & Milosavljevic, A. (2019). exceRpt: A Comprehensive Analytic Platform for Extracellular RNA Profiling. https://doi.org/10.1016/j.cels.2019.03.004
Srinivasan, S., Duval, M. X., Kaimal, V., Cuff, C., & Clarke, S. H. (2019). Assessment of methods for serum extracellular vesicle small RNA sequencing to support biomarker development. Journal of Extracellular Vesicles, 8(1). https://doi.org/10.1080/20013078.2019.1684425
Stephens, M. (2017). False discovery rates: a new deal. Biostatistics, 18(2), 275-294. https://doi.org/10.1093/BIOSTATISTICS/KXW041
Théry, C., Amigorena, S., Raposo, G., & Clayton, A. (2006). Isolation and Characterization of Exosomes from Cell Culture Supernatants and Biological Fluids. Current Protocols in Cell Biology, 30(1), 3.22.1-3.22.29. https://doi.org/10.1002/0471143030.CB0322S30
Théry, C., Witwer, K. W., Aikawa, E., Alcaraz, M. J., Anderson, J. D., Andriantsitohaina, R., Antoniou, A., Arab, T., Archer, F., Atkin-Smith, G. K., Ayre, D. C., Bach, J. M., Bachurski, D., Baharvand, H., Balaj, L., Baldacchino, S., Bauer, N. N., Baxter, A. A., Bebawy, M., ... Zuba-Surma, E. K. (2018). Minimal information for studies of extracellular vesicles 2018 (MISEV2018): a position statement of the International Society for Extracellular Vesicles and update of the MISEV2014 guidelines. Https://Doi.Org/10.1080/20013078.2018.1535750, 7(1). https://doi.org/10.1080/20013078.2018.1535750
Thygesen, K., Alpert, J. S., Jaffe, A. S., Chaitman, B. R., Bax, J. J., Morrow, D. A., White, H. D., Thygesen, K., Alpert, J. S., Jaffe, A. S., Chaitman, B. R., Bax, J. J., Morrow, D. A., White, H. D., Mickley, H., Crea, F., van de Werf, F., Bucciarelli-Ducci, C., Katus, H. A., ... Corbett, S. (2019). Fourth universal definition of myocardial infarction (2018). European Heart Journal, 40(3), 237-269. https://doi.org/10.1093/eurheartj/ehy462
Tompkins, B. A., Balkan, W., Winkler, J., Gyöngyösi, M., Goliasch, G., Fernández-Avilés, F., & Hare, J. M. (2018). IMPACT: Preclinical studies of cell therapy for human disease. Circulation Research, 122(7), 1006. https://doi.org/10.1161/CIRCRESAHA.117.312486
Wang, Q.-Z., Zhao, Z.-L., Liu, C., & Zheng, J.-W. (2021). Exosome-derived miR-196b-5p facilitates intercellular interaction in infantile hemangioma via down-regulating CDKN1B. Annals of Translational Medicine, 9(5), 394-394. https://doi.org/10.21037/ATM-20-6456
Wang, S., Aurora, A. B., Johnson, B. A., Qi, X., McAnally, J., Hill, J. A., Richardson, J. A., Bassel-Duby, R., & Olson, E. N. (2008). The Endothelial-Specific MicroRNA miR-126 Governs Vascular Integrity and Angiogenesis. Developmental Cell, 15(2), 261-271. https://doi.org/10.1016/j.devcel.2008.07.002
WHO-Cardiovascular diseases (CVDs). (2017). http://www.who.int/en/news-room/fact-sheets/detail/cardiovascular-diseases-(cvds)
Zhang, J., Chu, L. F., Hou, Z., Schwartz, M. P., Hacker, T., Vickerman, V., Swanson, S., Leng, N., Nguyen, B. K., Elwell, A., Bolin, J., Brown, M. E., Stewart, R., Burlingham, W. J., Murphy, W. L., & Thomson, J. A. (2017). Functional characterization of human pluripotent stem cell-derived arterial endothelial cells. Proceedings of the National Academy of Sciences of the United States of America, 114(30), E6072-E6078. https://doi.org/10.1073/PNAS.1702295114
Zhou, H., Yuen, P. S. T., Pisitkun, T., Gonzales, P. A., Yasuda, H., Dear, J. W., Gross, P., Knepper, M. A., & Star, R. A. (2006). Collection, storage, preservation, and normalization of human urinary exosomes for biomarker discovery. Kidney International, 69(8), 1471-1476. https://doi.org/10.1038/SJ.KI.5000273

## Claims

1. An isolated population of pro-angiogenic and/or pro-neovasculogenic extracellular vesicles (EVs) and/or their contents thereof, obtainable from pluripotent stem-cell derived endothelial cell products.

2. The isolated population of pro-angiogenic and/or pro-neovasculogenic EVs and/or their contents thereof according to claim 1, for use in therapy.

3. A pharmaceutical composition comprising an isolated population of pro-angiogenic and/or pro-neovasculogenic EVs and/or their contents thereof according to claim 1, optionally comprising a pharmaceutically acceptable carrier and/or excipient therefor.

4. A medical device coated with an isolated population of pro-angiogenic and/or pro-neovasculogenic EVs and/or their contents thereof according to claim 1

5. The isolated population of pro-angiogenic and/or pro-neovasculogenic EVs and/or their contents thereof, the pharmaceutical composition, or medical device according to any preceding claim for use in promoting angiogenesis and/or neovascularisation, in a subject in need thereof.

6. The isolated population of pro-angiogenic and/or pro-neovasculogenic EVs and/or their contents thereof, the pharmaceutical composition, or medical device according to claim 5, for use in the treatment of ischemic tissue disorders, cardiovascular diseases such as pulmonary hypertension and/or wound healing.

7. The isolated population of pro-angiogenic and/or pro-neovasculogenic EVs and/or their contents thereof, or the pharmaceutical composition according to claim 4, for use in an amount less than or equal to 50, 25, 10, 5, 2, or 1 EV/cell

8. A method of isolating EVs from endothelial cells derived from pluripotent stem cells, the method comprising:
a) subjecting a fluid comprising EVs produced by endothelial cells derived from pluripotent stem cells to ultrafiltration, in order separate EVs from larger molecular weight material and obtain an EVs enriched fluid; and
b) subjecting the EV enriched fluid to size-exclusion chromatography (SEC) in order to obtain a fluid fraction comprising isolated EVs.

9. The method according to claim 8 wherein ultrafiltration comprises employing a filter with a 100KDa molecular weight cut-off.

10. The method according to either of claims 8 and 9, wherein SEC is conducted using sephacryl, sephadex, superose, superdex or sepharose chromatography media, especially sepharose, such as sepharose CL-6B.

11. An isolated population of pro-angiogenic and/or pro-neovasculogenic extracellular vesicles (EVs) and/or their contents thereof prepared according to the method of claims 8 - 10, for use in promoting angiogenesis and/or neovascularisation.

12. The isolated population of pro-angiogenic and/or pro-neovasculogenic extracellular vesicles (EVs) and/or their contents thereof according to claim 11 for use in the treatment of ischemic tissue disorders, cardiovascular diseases such as pulmonary hypertension and/or wound healing.
